(19)

Europäisches
Patentamt
European
Patent Office
Office européen
des brevets

(11) **EP 3 664 704 B1**

(12) **EUROPEAN PATENT SPECIFICATION**

(45) Date of publication and mention
of the grant of the patent:
**19.04.2023   Bulletin 2023/16**

(21) Application number: **18746957.2**

(22) Date of filing: **02.08.2018**

(51) International Patent Classification (IPC):
***A61B 5/024*** *(2006.01)*      ***A61B 5/00*** *(2006.01)*

(52) Cooperative Patent Classification (CPC):
**A61B 5/0033; A61B 5/02416**

(86) International application number:
**PCT/EP2018/071072**

(87) International publication number:
**WO 2019/030124 (14.02.2019 Gazette 2019/07)**

(54) **DEVICE, SYSTEM AND METHOD FOR DETERMINING A PHYSIOLOGICAL PARAMETER OF A SUBJECT**

VORRICHTUNG, SYSTEM UND VERFAHREN ZUR BESTIMMUNG EINES PHYSIOLOGISCHEN
PARAMETERS EINES SUBJEKTS

DISPOSITIF, SYSTÈME ET PROCÉDÉ PERMETTANT DE DÉTERMINER UN PARAMÈTRE
PHYSIOLOGIQUE D'UN SUJET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **08.08.2017   EP 17185308**

(43) Date of publication of application:
**17.06.2020   Bulletin 2020/25**

(73) Proprietors:
• **Koninklijke Philips N.V.**
**5656 AG Eindhoven (NL)**
• **Technische Universiteit Eindhoven**
**5612 AE Eindhoven (NL)**
Designated Contracting States:
**NL**

(72) Inventors:
• **WANG, Wenjing**
**5656 AE Eindhoven (NL)**

• **DE HAAN, Gerard**
**5656 AE Eindhoven (NL)**
• **DEN BRINKER, Albertus, Cornelis**
**5656 AE Eindhoven (NL)**

(74) Representative: **Philips Intellectual Property &
Standards**
**High Tech Campus 52**
**5656 AG Eindhoven (NL)**

(56) References cited:
**WO-A1-2011/042839      WO-A1-2014/180660
WO-A1-2018/029123      WO-A2-2011/127487
US-A1- 2014 276 099**

• **ANDREIA V. MO?O ET AL: "Motion robust
PPG-imaging through color channel mapping",
BIOMEDICAL OPTICS EXPRESS, vol. 7, no. 5, 6
April 2016 (2016-04-06), page 1737, XP055285676,
United States ISSN: 2156-7085, DOI:
10.1364/BOE.7.001737**

**Description**

FIELD OF THE INVENTION

The present invention relates to the fields of medical technology and camera-based vital signs monitoring using remote photoplethysmography (rPPG). In particular, the present invention relates to a device and system for determining a physiological parameter of a subject. The present invention further relates to a corresponding method and a computer program for carrying out said method.

BACKGROUND OF THE INVENTION

Vital signs of a person, for example the heart rate (HR), the respiration rate (RR) or the arterial blood oxygen saturation, serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation can be determined.

Recently, non-contact, remote PPG (rPPG) devices (also called camera rPPG device herein) for unobtrusive measurements have been introduced. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications. This technology particularly has distinct advantages for patients with extreme skin sensitivity requiring vital signs monitoring such as Neonatal Intensive Care Unit (NICU) patients with extremely fragile skin, premature babies, or patients with extensive bums.

Verkruysse et al., "Remote plethysmographic imaging using ambient light", Optics Express, 16(26), 22 December 2008, pp. 21434-21445 demonstrates that photoplethysmographic signals can be measured remotely using ambient light and a conventional consumer level video camera, using red, green and blue (RGB) color channels.

A drawback of camera-based vital signs monitoring using remote photoplethysmography, is that often only limited region-of-interest (ROI) in the camera images provides valuable vital sign information. The region-of-interest has to be selected in the image frames and tracked over time. For example, face detection and face tracking can be used to identify and track a region-of-interest such as the cheeks or forehead of a subject. This selection is a process that comes with inaccuracies. A consequence of these inaccuracies is that the typical region from which a PPG signal has to be extracted partially comprises non-skin pixels that may corrupt the extracted PPG signal when these non-skin pixels are combined with skin pixels.

WO 2014/180660 A1 relates to a device and method for obtaining a vital sign of a subject despite motion of the subject, in particular for discriminating a vital sign such as a respiratory information signal from noise in a projection based vital signs registration. The disclosed device comprises an interface for receiving a set of image frames of a subject, an analysis unit for determining the amount of direction changes and/or the time distances between direction changes within a region of interest in a subset of image frames comprising a number of image frames of said set, a direction change indicating a change of the direction of motion appearing within said region of interest, an evaluation unit for determining if said region of interest within said subset of image frames comprises a vital sign information and/or noise by use of the determined amount of direction changes and/or the time distances for said subset of image frames, and a processor for determining the desired vital sign of the subject from said region of interest within said subset of image frames if it is determined that said region of interest within said subset of image frames comprises a vital sign information.

WO 2018/029123 A1, which forms part of the prior art within the terms of Article 54(3) EPC, relates to a device, system and method for use in blood oxygen saturation measurement of a subject. To enable blood oxygen saturation measurements with improved reliability, a processing device is presented comprising an input for receiving first and second detection data of a tissue region of the subject, said first detection data being data acquired over time by detecting radiation at a first wavelength ($\lambda$1) and at a second wavelength ($\lambda$2) received from said tissue region; said second detection data being data acquired over time by detecting radiation at the first wavelength and at the second wavelength received from said tissue region in response to coherent light at the first wavelength and coherent light at the second wavelength being emitted towards the tissue region; a PPG unit for deriving, from said first detection data, a first PPG signal indicative of an absorption of light within the tissue region at the first wavelength, and a second PPG signal indicative of an absorption of light within the tissue region at the second wavelength; a flow unit for deriving, from said second detection data, a first flow signal indicative of a flow of light scattering particles within the tissue region probed at the first wavelength, and a second flow signal indicative of a flow of light scattering particles within the tissue region probed at the second wavelength; and a processing unit for correcting said PPG signals based on said flow signals and/or for providing a feedback signal based on a comparison of the first and second flow signals.

WO 2011/042839 A1 discloses method and system for obtaining a first signal for analysis to characterize at least one periodic component thereof.

US 2014/0276099 A1 discloses a device and method for determining vital signs of a subject.

Moço et al., "Motion robust PPG-Imaging through color channel mapping", Biomedical Optics Express, vol. 7, no. 5, pages 1737-1754, April 2016, relates to motion robust PPG-Imaging through color channel mapping.

WO 2011/127487 A2 discloses a further method and system for measurement of physiological parameters.

SUMMARY OF THE INVENTION

It would be advantageous to provide an improved device, system and method for determining a physiological parameter of a subject. In particular, it would be advantageous to improve the extraction of a physiological parameter from a polluted region-of-interest comprising non-skin pixels that pollute the region-of-interest.

To better address one or more of these concerns, in a first aspect of the present invention a device for determining a physiological parameter of a subject is presented, the device comprising:

- an interface for receiving image data of a scene, said image data comprising a time-sequence of image frames; and
- a processor for processing said image data, wherein the processor is configured to perform the steps of:
- weighting pixel values of the image frames by reducing a weight of non-skin pixels relative to skin pixels;
  determining, for each of said image frames, a first statistical parameter value indicative of a statistical dispersion of pixel values of said image frame;
- concatenating said first statistical parameter values over time based on the time-sequence of the corresponding image frames to obtain a first candidate signal;
- extracting a physiological parameter of the subject based on said first candidate signal.

In a further aspect of the present invention a system for determining a physiological parameter of a subject is presented, the system comprising:

- an imaging unit configured to acquire image data of a scene, said image data comprising a time-sequence of image frames; and
- a device for determining a physiological parameter of a subject as described above based on the acquired image data.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium can have similar and/or identical preferred embodiments as the claimed device, in particular as defined in the dependent claims and as disclosed herein.

The inventors have found that a signal quality of a PPG or candidate signal can be improved in particular in case where many non-skin pixels pollute the image frame or a region-of-interest therein or if a weighting map is not fully accurate. In other words, a typical region from which the candidate signal is extracted comprises skin pixels as well as non-skin pixels that may corrupt an extracted physiological parameter when they are combined with skin-pixels. The inventors have recognized that problems and disadvantages of the prior art relating to an averaging of pixels can be mitigated by computing a statistical property characterizing or indicative of a statistical dispersion, such as the variance or standard deviation, of pixels of the image frame and using this statistical parameter value in determining a candidate signal based on which a physiological parameter value of the subject is extracted. Hence, it has been found that evaluating a statistical parameter value indicative of a statistical dispersion of pixel values of said image frame can provide a superior result in case of pollution due to non-skin pixels.

In the following, some terms which are used throughout the application, shall be briefly explained and defined:

As used herein, a physiological parameter of the subject can refer to a physiological parameter indicative of a vital sign of the subject, such as a pulse, respiration rate or blood oxygen saturation of the subject.

As used herein, the image data of the scene can in particular refer to video data acquired by an (RGB) video camera. The images can represent a scene comprising at least portions of the subject's skin. The image data can refer to at least some of the image frames forming a video signal such as an RGB video signal, monochrome video signal, range imaging data or IR (infrared) video, optionally comprising one or more channels, in particular channels corresponding to different wavelengths. For example, a pixel can carry multiple color channels. In case of multiple channels, the dispersion metric can be computed for each channel independently. A plurality of candidate signals can be determined separately based on different channels of the image data. Alternatively, one candidate signal can be determined based on a plurality of different channels of the image data. The extraction of the physiological parameter of the subject can be based on one or more of said candidate signals.

As used herein, a statistical parameter value can refer to a statistical measure indicative of values of pixels of an image frame. The first statistical parameter value can be (descriptive of or correspond to) a statistical dispersion (value) of pixel values of an image frame. In particular, the statistical parameter value may not be determined as a product with a projection of an image frame or pixels thereof. Statistical dispersion (also called variability, scatter, or spread) can be indicative of an extent to which a distribution is stretched or squeezed. Common examples of measures of statistical dispersion are the variance, standard deviation, and interquartile range. The candidate signal can be determined by concatenating statistical parameter values of the corresponding image frames over time. For example, for each of the frames a standard deviation value of the pixel values of this image frame (or a portion thereof e.g. identified as the region-of-interest in an optional pre-processing step) is determined and the standard deviation values of subsequent image frames form the candidate signal over time.

The extraction of the physiological parameter of the subject based on the first candidate signal can be performed using known techniques. Exemplary techniques include but are not limited to evaluating a fixed weighted sum over candidate signals of different wavelength channels (RGB, IR), blind source separation techniques advantageously involving both candidate signals such as blind source separation based on selecting the most pulsatile independent signal, principal component analysis (PCA), independent component analysis (ICA), CHROM (chrominance-based pulse extraction), POS (wherein the pulse is extracted in a plane orthogonal to a skin-tone vector), PBV method (which uses a predetermined signature of a blood volume pulse vector), or APBV (an adaptive version of the PBV-method, also allowing blood oxygen saturation monitoring). It should be noticed that optional pre-processing may be applied to the candidate signals, such as e.g. correction of gains of one or more channels, to further improve the performance.

The processor can optionally be configured to perform additional steps such as normalizing a candidate signal, e.g. by dividing it by its (moving window) temporal mean, taking its logarithm and/or removing an offset. Moreover, the processor can optionally perform image (pre-)processing steps such as weighting pixels by a weighting map, resizing, rescaling, resampling or cropping of the (weighted) image frames. A (weighted) image frame as used herein can may thus also refer to such a (pre-)processed (weighted) image frame. Optionally, the solution proposed herein of using a statistical parameter indicative of a statistical dispersion of pixel values can also be applied to only a portion of the image frame. Optionally, said portion of the image may be identified by a (coarse) region-of-interest selection and/or tracking (pre-)processing step. An advantage of this combination is that the accuracy and/or robustness can be further improved.

According to an embodiment, said first statistical parameter value is indicative of at least one of a standard deviation, a variance, mean absolute difference, median absolute difference and/or an interquartile range. Hence, a statistical parameter value indicative of a statistical dispersion is evaluated instead of the conventional approach of averaging pixels (i.e. evaluating a central tendency metric) in a region-of-interest.

Optionally, the processor can be configured to perform the steps of:

- determining, for each of said image frames, a second statistical parameter value indicative of a central tendency of pixel values of said image frame;
- concatenating said second statistical parameter values over time based on the time-sequence of the corresponding image frames to obtain a second candidate signal; and
- extracting a physiological parameter of the subject based on said first and/or second candidate signal. It has been found that by determining a first statistical parameter value over time indicative of a statistical dispersion of pixel values provides an advantageous candidate signal in case of pollution by non-skin pixels in the image frame (or a region-of-interest selected therein). On the other hand, in case of non-skin pixels below a predetermined threshold, the evaluation of a central tendency of pixel values of the image frame, such as a mean or average of the pixel values, can provide improved performance. By extracting candidate signals using both techniques, or both statistical metrics, during the extraction step the candidate signal providing the best signal quality can be evaluated.

In a refinement, the processor can be configured to extract said physiological parameter of the subject based on the second candidate signal, wherein the extraction of the physiological parameter of the subject based on the second candidate signal is further supported by the first candidate signal. An advantage of this embodiment is that both the first candidate signal (dispersion-based) and the second candidate signal (central-tendency-based) are taken into account, thereby providing an improved accuracy. Instead of directly extracting the physiological parameter from the first candidate signal, the (dispersion-based) first candidate signal may assist or can be used as a reference in extracting the physiological parameter from the (central tendency -based) second candidate signal, in particular to clean up the second candidate signal. This is particularly advantageous in case the first candidate signal comprises e.g. amplitude distortions. Nevertheless the first candidate signal may support the extraction of the physiological parameter of the subject. A plurality of first and second candidate signals can be determined separately based on different channels of the image data. For example, assuming three color channels (RGB) and one candidate signal for each of the color channels for both the first and the second candidate signals, respectively, a (6-dim) signal matrix over time comprising [Rm(t),Gm(t),Bm(t),Rs(t),Gs(t),Bs(t)] can be generated, wherein RGB denote the color channels and the indices s and m the first candidate signals (e.g. determined based on a standard deviation as indicated by the index s) and second candidate signals (e.g. determined based on a mean as indicated by the index m), respectively, for the different color channels. Hence, a plurality of separate first and second candidate signals can be determined for different (color) channels. The signal matrix can be provided as an input to a MSSA (Multi-channel Singular Value Analysis), but only the output (i.e., reconstructed signals) of one or more of the channels corresponding to the second candidate signals (for instance the first three channels) are used to extract the physiological parameter. In this example, the dispersion-based first candidate signals (Rs,Gs,Bs) may thus serve as a reference to drive a signal decomposition and component grouping of the central-tendency-based second candidate signals (Rm,Gm,Bm), thereby further improving the accuracy.

Optionally, the processor can be further configured to determine the first statistical parameter value based on a logarithm of pixel values. An advantage of this embodiment is that the log-conversion enables a correction of relative sign of components. Relative pulsatilities in different wavelength channels can be fixed since the physiological parameter signal is expected to be proportional to a pixel DC-value; thereby making use of a multiplicative phenomenon. Optionally a small bias may be added to prevent log(0) resulting in minus infinity. In addition or in the alternative pixel values may be projected e.g. on fixed axis, for example by calculating: 2log(G)-log(R)-log(B), and then computing a dispersion metric based thereon.

Optionally, the processor can be further configured to perform a preprocessing step of selecting a region-of-interest by selecting pixels of an image frame (based on a local property of the image) indicative of a skin of the subject and to determine said first statistical parameter value indicative of a statistical dispersion based on pixel values from said region-of-interest. An advantage of this embodiment is that the accuracy can be further improved. A region-of-interest may be determined based on a local property of the image e.g. a color, brightness, local texture, depth, and/or temperature (depending on the image data provided by the imaging unit). Optionally, feature-based face detection can be used. For instance, a Viola-Jones face detector can be applied. In addition or in the alternative, the processor can be configured to perform a pre-processing step by applying a set of different weighting maps to the image, as will be described in more detail further below.

The processor is configured to weight pixel values of the images frames by reducing a weight of non-skin pixels relative to skin pixels. An advantage of this embodiment is that the performance can be further improved, since non-skin pixels are given a lower weight. It shall be understood that the decision between skin pixels and non-skin pixels may not be a binary decision. Instead a likelihood of being a (non-) skin pixel can be evaluated. Hence the pixel values can be weighted based on the likelihood of a pixel bring a (non-) skin pixel. Optionally, a set of weighting maps can be applied.

Optionally, said step of extracting the physiological parameter can comprise correction, in particular based on a principal component of at least pixels of a (weighted) image frame. An advantage of this embodiment is a further improved accuracy. For example, assuming that the image frame or a region-of-interest selected therein, includes some background that has a color different from the skin in the image frame or region-of-interest, then a Principal Component Analysis (PCA) of the pixel-values inside the image frame or region-of-interest can indicate a difference vector between these two colors as the first principal component. Since a pulse-induced color variation can modulate a dispersion-based metric by changing the length of this difference vector, relative pulsatilities in the different colors can be different compared to using a central tendency metric such as the mean, where the relative pulsatilities result as variations of the skin-vector with respect to e.g. black. Consequently, a correction can advantageously be applied to achieve e.g. the same or similar relative pulsatilities when using a dispersion metric, by dividing the obtained (relative, i.e., after normalization or taking the logarithm) pulsatilities in the color-channels by the first principal component vector. The corrected relative pulsatilities are now advantageously similar to the relative pulsatilities obtained by a central tendency metric on a skin-only region of interest. An advantage of this approach is that the same pulse-extraction methods can be used. Several different ways of correction can be used, optionally even in parallel: (i) Use of principal component analysis (PCA) to correct a relative amplitude of different color channels (or candidate signals obtained based on different color channels). Alternatively, a PBV vector of PBV, a set of PBV-vectors in ABPV, or a projection-axes of CHROM/POS can be corrected. Thereby, prior-related rPPG methods can be used. (b) Use of weighting maps, in particular in accordance with the first aspect of the present disclosure, to suppress non-skin pixels (similar to "pulling non-skin pixels to black"). Thereby, prior-related rPPG methods can be used. (c) Use the blind source separation (BSS) extraction. Thereby, amplitude-correction or pixel-weighting are not necessarily needed. (d) Combine the multi-wavelength images into a single-wavelength image (for example by 2log(G)-log(R)-log(B)) in a preprocessing step and then use a dispersion metric to combine spatial pixels.

In a refinement, the extracting of the physiological parameter can be based on a pulse-blood-volume (PBV) extraction method and wherein said correction based on a principal component value can be applied to a PBV-signature vector.

Optionally, the processor can be configured to determine at least the first candidate signal and a second candidate signal and the step of extracting the physiological parameter of the subject can further comprise the step of selecting at least one candidate signal based on a quality metric. For example, the candidate signal having the higher signal-to-noise ratio (SNR) may form the basis for extracting the physiological parameter of the subject. In addition or in the alternative, a flatness of a spectrum, a height of a spectral peak in a (normalized) spectrum or an entropy of the spectra of a candidate signals may be evaluated. The evaluation can include a comparison with a predetermined threshold. The second candidate signal may be determined based on a different statistical metric, for example, a second statistical parameter value indicative of a central tendency of pixel values of said image frame or a statistical parameter value indicative of a statistical dispersion of pixel values of said image frame.

Optionally, the processor can be further configured to determine at least the first candidate signal and a second candidate signal and to apply a blind source separation technique to the candidate signals to obtain independent signals and to select at least one of said independent signals based on a quality metric. Examples of blind source separation techniques are principal component analysis (PCA) and independent component analysis (ICA). Quality metrics can be used as described above. The second candidate signal may be determined based on a different statistical metric, for example, a second statistical parameter value indicative of a central tendency of pixel values of said image frame or a statistical parameter value indicative of a statistical dispersion of pixel values of said image frame.

Optionally, the processor can be configured to determine at least the first candidate signal and a second candidate signal and to combine at least two of said candidate signals, in particular in the frequency domain, in particular wherein different relative weights may be given to individual spectral components of said candidate signals, in particular to combine different spectral components of said candidate signals in individually optimal ways. Various options exist to determine the optimal weights per spectral components, e.g. they may be based on the amplitude of the spectral components. Optionally, a combination signal may be synthesized based on the candidate signals. In an embodiment, blind-source-separation may work on complete candidate signals in the time domain (i.e. providing a linear combination), whereas the spectral weighting may work on the spectral components in the frequency domain (thereby providing a non-linear combination). In other words, the processor can be configured to combine at least two of the candidate signals by means of a linear combination or a non-linear combination to obtain a combination signal, wherein the processor is further configured to extract the physiological parameter of the subject based on the combination signal. An advantage of such a combination is that the use of the information content of the candidate signals can be further improved.

Optionally, said image data can comprise at least two channels, in particular a first channel indicative of a first wavelength interval and a second channel indicative of a second wavelength interval. An advantage of this embodiment is that this enables even more precise analysis than using a single channel, since more parameters are available. For example first the different channels, using the same statistical metrics, may be combined to candidate signals for each of the different statistical metrics, and next combined, either in the time domain (e.g. using BSS), or in the frequency domain allowing different combination weights per spectral component. Alternatively, spectral weighting and combining of different wavelength channels may be reversed. As an example of multiple wavelength channels, different color channels of an RGB can be evaluated. In addition or in the alternative at least one of said channels may be an infrared (IR), near infrared (NIR) or thermal imaging channel. For example, one or more different infrared channels may be evaluated, for example indicative wavelength intervals comprising a wavelength of 775 nm, 800 nm and/or 905 nm, respectively. In addition or in the alternative, at least one of said channels can be indicative of a depth or range acquired by a range imaging technique such as using time of flight (TOF) camera, using structured light or a stereo camera. For example, the image may be segmented taking into account a temperature (warm skin) or a distance (expected distance of skin or background to camera).

Hence, referring again to the system for determining a physiological parameter of the subject, the imaging unit configured to acquire image data of the scene can comprise one or more of an RBG (video) camera, a temperature camera and/or a depth camera.

It shall be understood that the processor (also referred to as processing unit) can be implemented as a single entity such as a microcontroller, field programmable gate array (FPGA) or may also be implemented as a distributed processing device comprising a plurality of separate processing entities or even a cloud-based solution. The processor may also be shared with other applications. The interface can be wired or wireless interface for receiving said image data. The interface can be an interface of the processor. The proposed device may also refer to a signal processing device. Advantageously, the proposed device or system may be co-integrated in a patient monitor or hospital information system.

BRIEF DESCRIPTION OF THE DRAWINGS

These and other aspects of the invention will be apparent from and elucidated with reference to the embodiments described hereinafter. In the following drawings

Fig. 1 shows a schematic diagram of a system in accordance with an embodiment of the present invention;
Fig. 2 shows a flow chart of a method according to a first aspect of the present disclosure;
Fig. 3 shows a flow chart regarding processing of an individual image frame in accordance with said first aspect;
Fig. 4 shows a diagram regarding the processing of a sequence of image frames in accordance with said first aspect;
Fig. 5 shows a flow chart of a method according to a second aspect of the present disclosure;
Fig. 6 shows a second flow chart in accordance with said second aspect of the present disclosure;
Fig. 7 shows a block diagram combining advantageous aspects of the first and the second aspect of the present disclosure;
Fig. 8 shows a diagram of images corresponding to different processing steps;
Fig. 9 shows a diagram of images and weighting maps over time;
Fig. 10 shows a diagram regarding extraction of a vital sign parameter based on different regions of an image;
Fig. 11 shows a comparison of a conventional ECG-based measurement and camera-based vital signs measurement according to the present disclosure;
Fig. 12 shows a graph of a signal quality of obtained candidate signals based on a statistical parameter indicative of a central tendency or statistical parameter indicative of a statistical dispersion versus the skin percentage in a region-of-interest; and
Fig. 13 shows a diagram regarding weighting of image frames;

DETAILED DESCRIPTION OF THE EMBODIMENTS

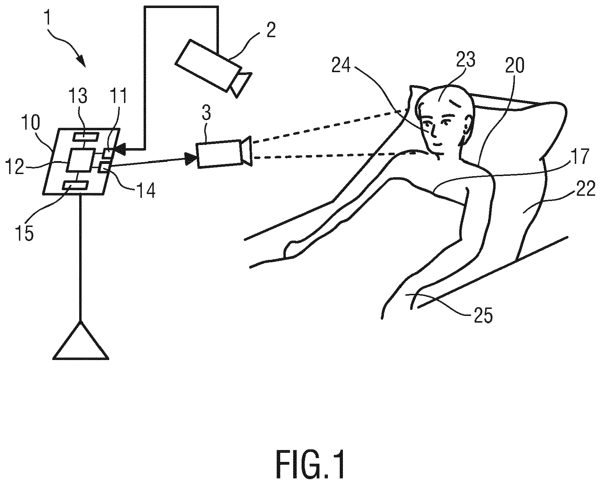

Fig. 1 shows a schematic diagram of a system 1 for determining a physiological parameter of a subject. The system 1 comprises an imaging unit 2 configured to acquire image data of a scene, said image data comprising a time-sequence of image frames; and a device 10 for determining a physiological parameter of a subject 20.

In this example shown in Fig. 1, the subject 20 is a patient lying in bed 22, for instance in a hospital or other healthcare facility, but may also be a neonate or premature infant, e.g. lying in an incubator, or a person at home or in a different environment, such as an athlete doing sports.

The imaging unit 2 may include a camera (also referred to as detection unit or remote PPG sensor) for acquiring an image data, said image data comprising a time-sequence of image frames. The image data can be indicative of reflected electromagnetic radiation, in particular in a wavelength range of visual and/or infrared light. The image data represents a scene, preferably comprising skin areas 23, 24, 25 of the subject 20 from which a physiological parameter of the subject 20 can be derived. Exemplary skin areas that are usually not covered by a blanket 26 or clothing are the forehead 23, the cheeks 24 or the hands or arms 25.

The image frames captured by the imaging may particularly correspond to a video sequence captured by means of an analog or digital photo sensor, e.g. in a (digital) camera. Such a camera usually includes a photo sensor-array, such as a CMOS or CCD image-sensor, which may also operate in a specific spectral range (visible, NIR) or provide information for different spectral ranges, particularly enabling the extraction of PPG signals. The camera may provide an analog or digital signal. The image frames include a plurality of image pixels having associated pixel values. Particularly, the image frames may include pixels representing light intensity values captured with different photosensitive elements of a photo sensor. These photosensitive elements may be sensitive in a specific spectral range (i.e. representing a specific color). The image frames include at least some image pixels being representative of a pulsatile region such as a skin portion of the person. Thereby, an image pixel may correspond to one photosensitive element of a photo-detector and its (analog or digital) output or may be determined based on a combination (e.g. through binning) of a plurality of the photosensitive elements.

The system 1 may further comprise a optional light source 3 (also called illumination source or light source or electromagnetic radiator), such as a lamp or LED, for illuminating/irradiating a region-of-interest, such as the skin areas 23, 24 of the patient's face (e.g. part of the cheek or forehead), with light, for instance in a predetermined wavelength range or ranges (e.g. in the red, green and/or infrared wavelength range(s)). The light reflected from the scene in response to said illumination is detected by the imaging unit or camera 2. In another embodiment no dedicated light source is provided, but ambient light is used for illumination of the subject scene. From the reflected light only light in a desired wavelength range (e.g. green and red or infrared light, or light in a sufficiently large wavelength range covering at least two wavelength channels) may be detected and/or evaluated. In case of using a thermal camera, radiation of the human body may be used directly.

The device 10 comprises an interface 11 for receiving image data of the scene, the image data comprising a time-sequence of image frames; and a processor 12.

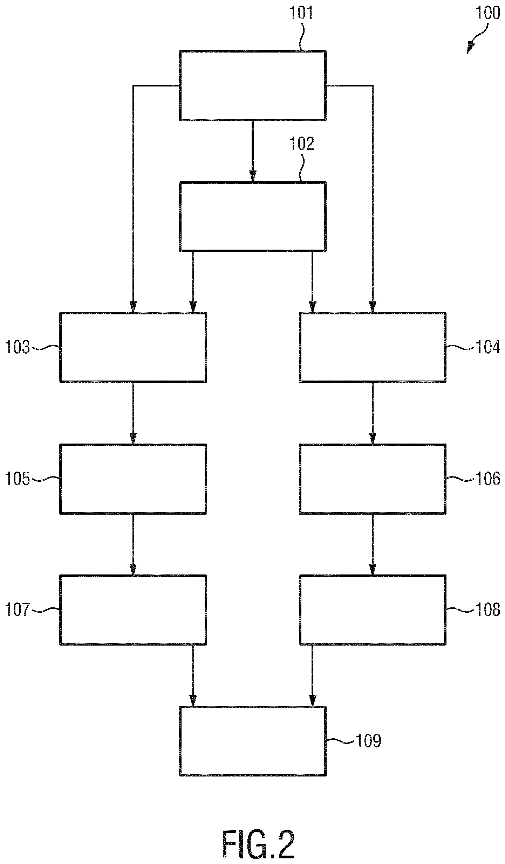

According to a first aspect of the present disclosure, the processor 12 is configured to perform at least some of the steps as described with reference to Fig. 2 to Fig. 4.

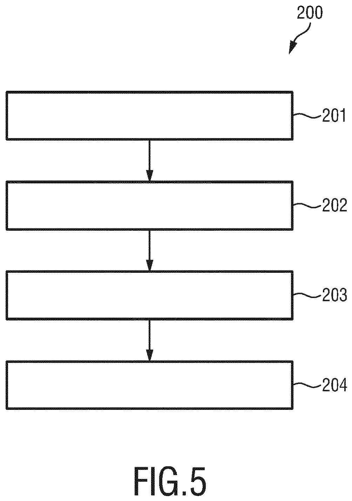

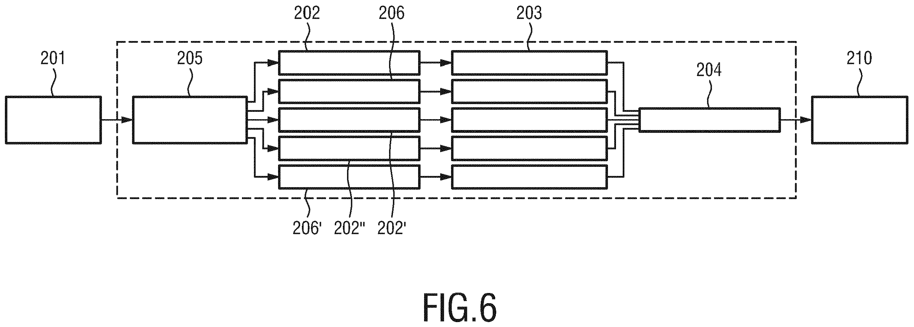

According to a second aspect of the present disclosure, the processor is configured to perform at least some of the steps as described with reference to Fig. 5 and Fig. 6.

The device 10 can comprise a memory or storage 13 that stores therein a computer program product or program code which causes at least one of said methods to be performed when carried out by the processor 12. The device 10 can further comprise an interface 14 for controlling another entity such as an external light source 3.

The device 10 can further comprise an interface 15 for displaying the extracted physiological parameter such as the pulse of the subject 20 and/or for providing medical personnel with an interface to change settings of the device 10, the camera 2, the light source 3 and/or any other parameter of the system 1. Such an interface 20 may comprise different displays, buttons, touchscreens, keyboards or other human machine interface means.

A system 1 as illustrated in Fig. 1 may, e.g., be located in a hospital, healthcare facility, elderly care facility or the like. Apart from the monitoring of patients, the solutions proposed herein may also be applied in other fields such as neonate monitoring, general surveillance applications, security monitoring or so-called lifestyle environments, such as fitness equipment, a wearable, a handheld device like a smartphone, or the like. The uni- or bidirectional communication between the device 10 and the imaging unit 2 may work via a wireless or wired communication interface. Other embodiments of the present invention may include a device 10, which is not provided stand-alone, but integrated into another entity such as for example the camera 2, a patient monitor, a hospital information system (HIS), cloud-based solution or other entity.

Remote photoplethysmography (rPPG) enables contactless monitoring of a physiological parameter of a subject such as monitoring of a cardiac activity by detecting pulse-induced subtle color changes of human skin surface for example using a regular RGB camera. In recent years, algorithms used for pulse extraction have matured but the additionally required means for full automation are much less developed, especially for the long-term monitoring. There are two conventional ways to automate an rPPG system. The first one (most commonly used) uses face detection, face tracking and skin selection. The second one uses dense video segmentation with local pulse estimation to find living skin pixels to initialize the measurement. However, neither is designed for long-term monitoring in real clinical applications such as sleep monitoring and neonatal monitoring. For instance the first approach is ipso facto not applicable to general body-parts (e.g., palm) or newborns. Furthermore, face detection may fail when the subject changes posture during sleep, when the camera registers the face under an unfavorable angle or when part of the face is covered by a blanket. The second approach needs spatio-temporally coherent local segments to create long-term time-tubes for pulse extraction and living-skin detection. Hence, this method is sensitive to local motion and computationally expensive. Essentially, living-skin detection and pulse extraction depend on each other. Regarding these conventional approaches, the common feature is that both include the region-of-interest (ROI) identification as an essential step prior to pulse extraction.

The inventors recognize that for vital signs monitoring, it is only required to extract a target signal indicative of a physiological parameter of the subject such as the pulse of the subject, as the output but it is not necessary to provide specifics of a location of a region-of-interest (ROI location). Hence, according to the first aspect of the present disclosure, there is proposed a method to directly extract a physiological parameter of the subject such as the pulse, even from a full video, eliminating the step of ROI localization and tracking.

The approach described with reference to the first aspect of the present disclosure is based on assumption that the DC-colors of skin and background are usually quite stable over time. Even though the spatial location of the subject may vary from image to image, as the subject can be anywhere in the image, the DC-colors of surfaces in the scene (including skin and background such as hospital bedding) will hardly change. Therefore, it is proposed to use e.g. the DC-color as a feature to automate the pulse extraction, rather than an ROI location. Hence, the proposal builds on the hypothesis that the background color and light source color remain stable at least in the (relatively short/sliding) time-window required for extracting the physiological parameter. At least in restricted applications such as a clinical setup it is further possible to manage the illumination by light sources and to control background color to have at least a short-term stable emission spectrum. Hence, it is suggested to exploit e.g. the DC-color as a spatial feature to differentiate objects in an image for pulse extraction, ignoring their locations or motions.

Fig. 2 shows a flow chart of a method for determining a physiological parameter of a subject according to the first aspect of the present disclosure. The method is denoted in its entirety by reference numeral 100.

In a first step 101, image data of a scene is received, said image data comprising a time-sequence of image frames. In the non-limiting embodiment described herein, the image data can be video data acquired by an RGB camera.

In a second step 102, a set of weighting maps is generated for each of said image frames. Each set of weighting maps comprises at least a first weighting map and a second weighting map for weighting pixels of the corresponding image frame.

In a third step 103, a first weighted image frame is determined by weighting pixels of the image frame (received in step 101) based on the first weighting map (determined in step 102). Correspondingly, in step 104, a second weighted image frame is determined by weighting pixels of the image frame (acquired in step 101) by weighting pixels of the image frame based on the second weighting map (also determined in step 102).

In step 105 a first statistical parameter value is determined based on the first weighted image frame as determined in step 103. Correspondingly in step 106 a second statistical parameter value is determined based on the second weighted image frame as determined in step 104.

The aforementioned steps 102-106 are repeated for each of the image frames comprised in the image data such that a sequence of first and second statistical parameter values is provided over time. The first statistical parameter values over time provided by step 105 are concatenated in step 107 based on the time-sequence of the corresponding image frames to obtain a first candidate signal. Correspondingly, the second statistical parameter values over time provided by step 108 are concatenated based on the time-sequence of the corresponding image frames to obtain a second candidate signal. It should be noted that the image data may comprise a plurality but not necessarily all image fames provided by the imaging unit.

In step 109, a physiological parameter of the subject is extracted based on said first and/or said second candidate signal. In the given non-limiting example, the physiological parameter can be a pulse of the subject. In particular, the first and/or second candidate signals may be selected based on a quality metric. For example the candidate signal providing the better signal to noise ratio in a frequency range of interest for pulse extraction, e.g., between 40bpm and 240bpm, may be selected and the physiological parameter extracted based thereon. Alternative quality metrics can be used.

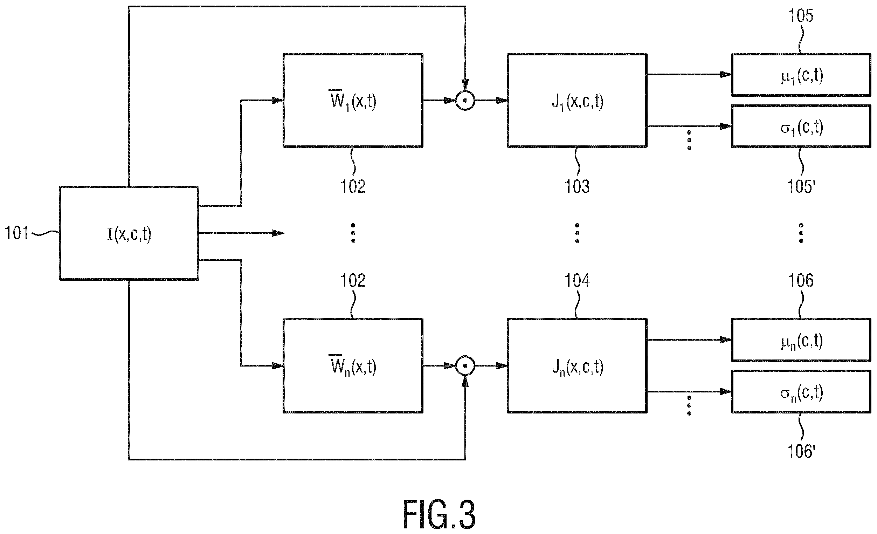

Fig. 3 illustrates the processing for a single image frame in more detail. The same reference numerals as in Fig. 2 will be used so that a correspondence between the figures can be established. As described above, in step 101 image data is received. In the following examples, the image data may refer to a video sequence registered by an RGB camera viewing a scene including a living skin. As used herein $I(x, c, t)$ denotes the intensity of a pixel at the location $x$ of an image in the (color) channel $c$ recorded at time $t$. Herein, the pixel location is given by an index $x$. Alternatively, reference could also be made to vertical and horizontal pixel coordinates within the image frame corresponding to said index. In a typical setup there can be $c = 1, 2, 3$ corresponding to the R-G-B (red, green blue) channels of a standard RBG camera. Optionally, down-sampling can be applied and the pixel $x$ can be created from a down-sampled version of the image to reduce both the quantization noise and the computational complexity. For example, a standard $640 \times 480$ or $1920 \times 1080$ pixel image can be down-sampled to e.g. $20 \cdot 20$ down-sampled pixels. The time $t$ can also denote a frame index which is related to the time by a recording rate of e.g. 20 frames per second (fps).

For each image frame, i.e. for each time $t$, a set of weighting maps $\overline{W}_1(x, t)...\overline{W}_n(x, t)$ is determined in step 102. Optionally, different weighting maps can be created for the different channels. However, as shown in Fig. 3, it is also possible to create a common weighting map taking into consideration the pixel values in two or more, preferably all, of the channels to fully exploit the information given in the channels of the image.

In step 103, a first weighted image frame $J_1(x, c, t)$ is determined by weighting pixels of the image frame $I(x, c, t)$ based on the first weighting map $\overline{W}_1(x, t)$. Correspondingly, the second weighted image frame is generated by weighting pixels of the (same) image frame $I(x, c, t)$ based on the second weighting map $\overline{W}_2(x, t)$ in step 104. It will be appreciated that the set of weighting maps can comprise a plurality of weighting maps 1...n. Hence, a plurality of corresponding weighted image frames $J_1 ... J_n$ can be determined accordingly.

For each of said weighted image frames, one or more statistical parameter values are extracted in a next stage. For example, for the first weighted image frame a first statistical parameter value can be extracted in step 105. In the given example, a mean or average value $\mu_1(c, t)$ is determined. Optionally, further statistical parameter values such as the standard deviation of the pixel values in the corresponding weighted image frame $J_i(x, c, t)$ can be determined as $\sigma_1(c, t)$ as indicated by reference numeral 105'. Correspondingly, one or more second statistical parameter values can be determined based on the second weighted image frame in steps 106 and 106'. The same applies to further optional weighted image frames. The sequence of first statistical parameter values obtained over time from a sequence of first weighted images over time can be concatenated over time to obtain a first candidate signal. Correspondingly, the sequence of second statistical parameter values obtained over time from the sequence of second weighted images over time can be concatenated over time to obtain a first candidate signal.

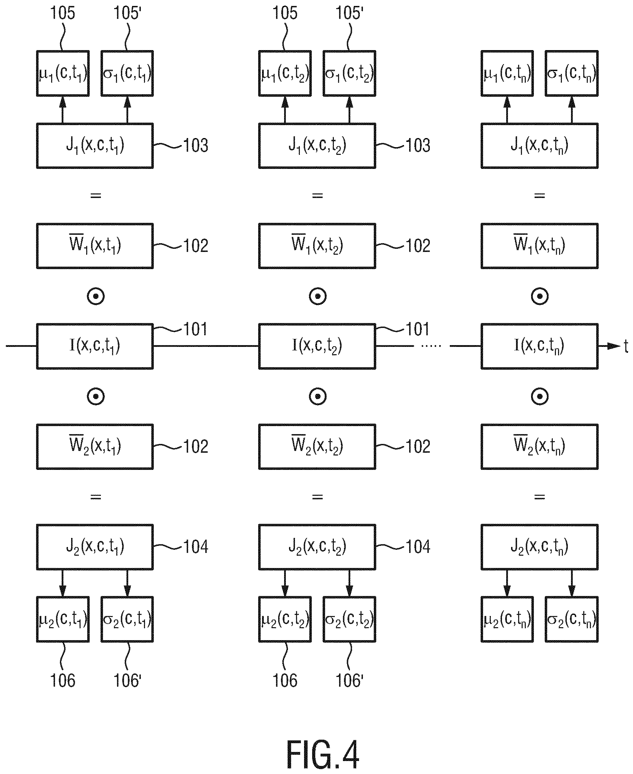

Fig. 4 illustrates the process over time. Therein, each 'column' of processing steps denotes one point in time $t_1...t_n$. In the given example four candidate signals are generated. In the given example, the first candidate signal can be obtained by concatenating the first statistical parameter values over time, here $\mu_1(c, t_1) ... \mu_1(c, t_n)$ based on the time-sequence of the corresponding image frames $I(x, c, t_1) ... I(x, c, t_n)$. Correspondingly, a second candidate signal can be obtained by concatenating said second statistical parameter values over time, here $\mu_2(c, t_1) ... \mu_2(c, t_n)$ based on the time-sequence of the corresponding image frames $I(x, c, t_1) ... I(x, c, t_n)$. Hence, in the given example, the first (and second) statistical parameter values refer to an average of the image frames that have been weighted by the first weighting map $\overline{W}_1$ (and the second weighting map $\overline{W}_2$), respectively.

Optionally, additional statistical parameter values can be obtained based on the first weighted image frame and/or the second image frame. In the given example, the standard deviation $\sigma$ as a dispersion related statistical parameter value is determined. Hence, a further candidate signal can be obtained by concatenating the statistical parameter values $\sigma_1 (c, t_1)...\sigma_1, (c, t_n)$ based on the time-sequence of the corresponding image frames I $(x, c, t_1)...$ I $(x, c, t_n)$ as obtained in the processing steps 105. The same can be applied to obtain a fourth candidate signal based on the processing steps 106' accordingly. Hence, the physiological parameter of the subject can be extracted based on one or more of said first, second, third and fourth candidate signals.

Figs. 5 and 6 refer to a second aspect of the present disclosure. Features described in conjunction with the second embodiment can be implemented in combination with features of the first aspect but may also be implemented independent thereof.

Referring to Fig. 5, in a first step 201, image data of a scene is received, said image data comprising a time-sequence of image frames.

In step 202, for each of said image frames, a first statistical parameter value indicative of a statistical dispersion of pixel values of said image frame is determined.

In step 203, said first statistical parameter values of the individual image frames are concatenated over time, based on the time-sequence of the corresponding image frame, to obtain a first candidate signal.

In step 204 a physiological parameter of the subject can be extracted based on said first candidate signal.

The inventors have found that a signal quality of a PPG or candidate signal can be improved in particular in case where many non-skin pixels pollute the image frame or a region-of-interest therein or if a weighting map (according to the first aspect) is not fully accurate. In other words, a typical region from which the candidate signal is extracted comprises skin pixels as well as non-skin pixels that may corrupt an extracted physiological parameter when they are combined with skin-pixels. The inventors have recognized that problems and disadvantages of the prior art relating to an averaging of pixels can be mitigated by computing a statistical property characterizing or indicative of a statistical dispersion, such as the variance or standard deviation, of pixels of the image frame and using this statistical parameter value in determining a candidate signal based on which a physiological parameter value of the subject is extracted. Hence, it has been found that evaluating a statistical parameter value indicative of a statistical dispersion of pixel values of said image frame can provide a superior result in case of pollution due to non-skin pixels.

Advantageously, this concept can be combined with the first aspect of the present disclosure. For example, referring again to Fig. 2, the statistical parameter value that is determined based on the first weighted image frame can be a statistical parameter value indicative of a statistical dispersion of pixel values of said weighted image frame. Correspondingly, the aspect of using a weighted image frame as an input can be used in the method as described with reference to Fig. 5 by providing for example a weighted image frame as an input in step 201.

Fig. 6 shows a second embodiment of processing steps according to the second aspect of the present disclosure. The same reference numerals as in Fig. 5 will be used so that a correspondence between the figures can be established.

In step 201, the image data of a scene is received via an interface, said image data comprising a time-sequence of image frames. In the embodiment shown in Fig. 6, there is provided an optional pre-processing step 205, wherein a region-of-interest can be selected. The region-of-interest can be selected using conventional processing techniques such as face detection and tracking. In the alternative, the aspect of using weighting maps can be implemented as a preprocessing step 205. The output of said preprocessing step may then be provided as an image frame to the subsequent processing steps.

In step 202, for each of said image frames (optionally after the preprocessing), a first statistical parameter value indicative of a statistical dispersion of the pixel values of such image frame is determined. The first statistical parameter value can be indicative of at least one of a standard deviation, a variance, mean absolute difference, median absolute difference and/or an interquartile range.

In a further optional step 206, for each of said image frames as obtained from step 205 or directly from step 201, a second statistical parameter value indicative of a central tendency of pixel values of said image frame can be determined. It has been found that by determining a first statistical parameter value over time indicative of a statistical dispersion of pixel values provides an advantageous candidate signal in case of pollution by non-skin pixels in the image frame (or a region-of-interest selected therein). On the other hand, in case of non-skin pixels below a predetermined threshold, the evaluation of a central tendency of pixel values of the image frame, such as a mean or average of the pixel values, can provide improved performance.

In step 203 said first statistical parameter values are concatenated over time based on the time-sequence of the corresponding image frames to obtain a first candidate signal. Correspondingly in step 207 said second statistical parameter values can be concatenated over time based on the time-sequence of the corresponding image frames to obtain a second candidate signal.

Optionally, one or more additional statistical parameter values indicative of a statistical dispersion of pixel values can be evaluated, for example, evaluating a standard deviation or variance as the first statistical parameter value and further evaluating an interquartile range as an additional processing step 202'. Correspondingly, additional second statistical parameter values indicative of other central tendency parameters can be evaluated. Correspondingly, the respective statistical parameter values can be concatenated over time to obtain additional candidate signals.

In step 204 a physiological parameter of the subject, such as the pulse of the subject, can be extracted based on said plurality of candidate signals. The same extraction methods as for the first aspect can be applied. The physiological parameter of the subject can be provided as an output in step 210.

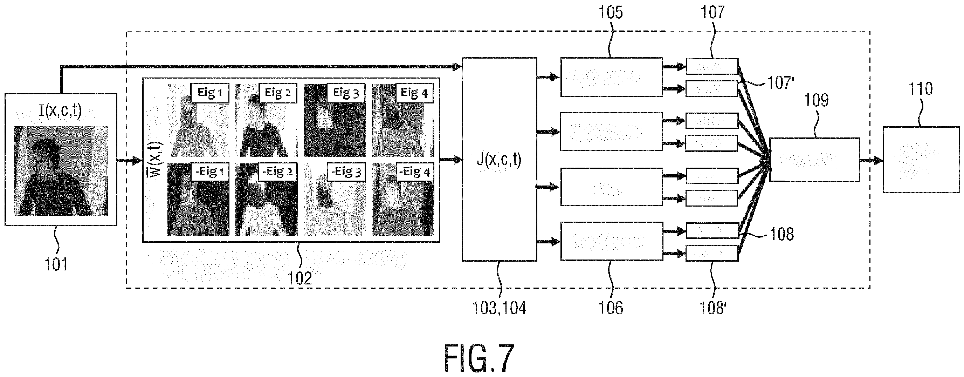

Fig. 7 now refers to an embodiment combining advantageous features of both the first and the second aspect of the present disclosure. In a given non-limiting example an RGB video is provided as the image data of a scene comprising a time-sequence image frames. Given a video registered by an RBG camera viewing the scene including a living-skin, I(x, c, t) is used to denote the intensity of a pixel at a location x of an image, in the channel c, recorded at time t. Herein, the channels c = 1, 2, 3 again correspond to the R-G-B channels of a standard RGB camera. The pixel x can optionally be created from a down-sampled version of the image to reduce both quantization noise and computational complexity, e.g., $20 \times 20$ down-sampled pixels or patches from an input image frame comprising $640 \times 480$ pixels. As used herein, the time t can again denote the frame index which corresponds to the time by the frame rate of herein 20 frames per second (fps).

In step 102 a set of weighting maps is created. Optionally a normalization step can be performed. Since it is aimed to combine the (down-sampled) pixels sharing the same color feature for pulse extraction through a set of weighting maps, the color-features can be normalized to be independent of the light intensity. The intensity of each pixel x can be normalized by:

$$\mathbf{I_n}(x, c, t) = \frac{\mathbf{I}(x, c, t)}{\sum_{c=1}^{3} \mathbf{I}(x, c, t)}, \qquad (1)$$

where $\mathbf{I_n}$ (x, c, t) denotes the locally normalized color values.

Next, $\mathbf{I_n}$ (t) can be used to generate multiple weighting maps wherein the pixels sharing similar normalized values are assigned to a similar weight. For example, spectral clustering as described in A. Y. Ng, M. I. Jordan, and Y. Weiss, "On spectral clustering: Analysis and an algorithm," in Advances In Neural Information Processing Systems, MIT Press, 2001, pp. 849-856, can be used to build a fully connected affinity/similarity graph for all the patches using $\mathbf{I_n}$ (t) and decompose it into uncorrelated subspaces, where a subspace can be used as an independent weighting mask to discriminate patches or pixels with different colors.

In line with this disclosure, the affinity matrix for all patch pixels in the t-frame can be built as:

$$A_{x,y}(t) = \|\mathbf{I_n}(x, t) - \mathbf{I_n}(y, t)\|_2, \qquad (2)$$

where $\|\cdot\|_2$ denotes the L2-norm (e.g. Euclidean distance), A denotes the symmetric and non-negative affinity matrix, where (down-sampled) pixels are pairwise connected. In a next step A can be decomposed into orthogonal (uncorrelated) subspaces using singular value decomposition (SVD):

$$\mathbf{A}(t) = \mathbf{u}(t) \cdot \mathbf{s}(t) \cdot \mathbf{u}^{\top}(t), \qquad (3)$$

where u (t) and s (t) denote the eigenvectors (u (t) · u (t)$^T$=1) and eigenvalues, respectively. Since each eigenvector describes a group of patches having a similar color feature, a number of K, preferably K top-ranked, eigenvectors can be used to create the weighting maps, where K can be defined, either automatically (using s(t)), or manually. Optionally, to fully exploit the eigenvectors, both the u (t) and -u (t) (i.e., the opposite direction) can be used to create the weighting vectors:

$$\mathbf{w}(t) = [\mathbf{u_1}(t), ..., \mathbf{u_K}(t), -\mathbf{u_1}(t), ..., -\mathbf{u_K}(t)], \qquad (4)$$

where $u_i$ (t) denotes the i-th (column) eigenvector and each column of w(t) represents an image weighting vector. A number of 2K weighting vectors can be generated by using the top-K eigenvectors. Optionally, since the weights in w(t) should preferably be non-negative and temporally stable, i.e., not driven by pulse, it can first be shifted by:

$$\hat{\mathbf{w}}_{\mathbf{i}}(t) = \mathbf{w}_{\mathbf{i}}(t) - \min(\mathbf{w}_{\mathbf{i}}(t)), \qquad (5)$$

where $w_i$ (t) denotes the i-th (column) weighting vector and **min** $(\cdot)$ denotes the minimum operator, and then optionally normalized by:

$$\bar{\mathbf{w}}_{\mathbf{i}}(t) = \frac{\hat{\mathbf{w}}_{\mathbf{i}}(t)}{\mathbf{sum}(\hat{\mathbf{w}}_{\mathbf{i}}(t))}, \qquad (6)$$

where **sum** $(\cdot)$ denotes the summation operator. This step is advantageous as it guarantees that the total weight for each frame can be the same, i.e. a normalization is applied. Hence, the total weight is time-stable and not driven/modulated by pulse. Each $\bar{w}_i(t)$ can be reshaped into a weighting map (with the same dimension as the image), denoted as $\bar{w}_i(t)$, and used to weight each channel of I(t) as:

$$\mathbf{J}_{\mathbf{i}}(x, c, t) = \bar{\mathbf{W}}_{\mathbf{i}}(x, t) \odot \mathbf{I}(x, c, t), \qquad (7)$$

where $J_i$ (x, c, t) denotes the x-th pixel in the c-th channel weighted by the i-th weighting map at time t. In Fig. 7 this step is denoted by 103, 104. The same reference numerals as in Fig. 2 to 4 are used so that a correspondence between the figures can be established.

In a next step 105, 106 each weighted image can be condensed into one or more spatial color representations given by a statistical parameter value. The respective statistical parameter values can be concatenated over time to provide a plurality candidate signals 107, 107', 108, 108'.

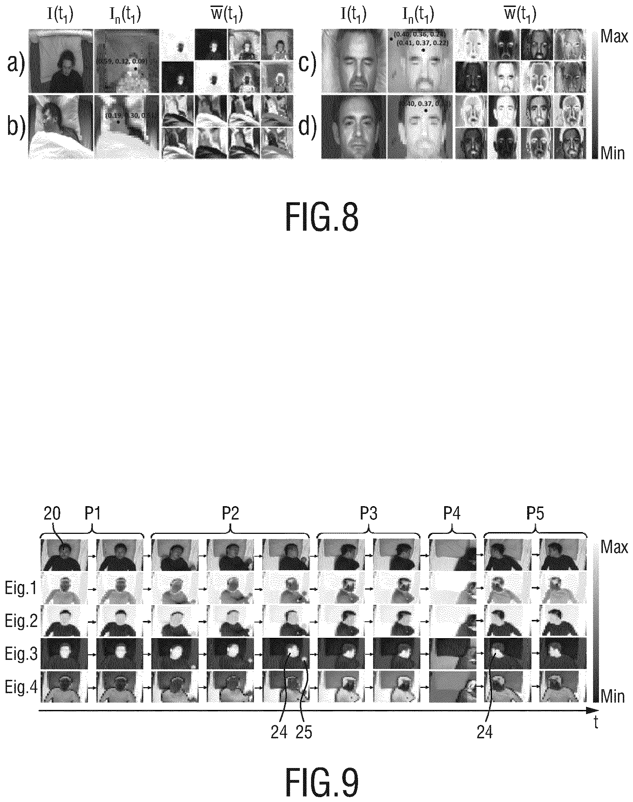

Fig. 8 exemplifies exemplary RGB images in Fig. 8(a) and (b) and Near Infrared (NIR) images in Fig. 8 (c) and (d) at a point-in time t along with their corresponding weighting maps based on the top-4 eigenvectors. The first image in Fig. 8(a) to (d) shows the intensity as described by I(x, c, t). The second image in Fig. 8(a) to (d) shows the normalized intensity $I_n$(x, c, t), as determined by equation (1) above. Since positive and negative eigenvectors are considered, eight weighting maps $\bar{W}_t$ are provided for each image.

It has been found that, in particular regarding the RGB images, both a visible light source color and background color can influence the weighting map. Also, from the normalized pixel values, as shown in the respective second images of Fig. 8(a) to (d) it can be seen that normalized pixel values of the skin can be very different in different lighting conditions. Such different conditions may be hardly modeled/learned off-line. Hence, an advantage of the approach disclosed herein is that no previous knowledge may be required. For the NIR images (in this exampled obtained at 675 nm, 800 nm and 905 nm), it has been found the visible light color does not influence the weighting maps substantially. It has been found that the skin can have similar reflections in the used NIR wavelengths, as opposed to the RGB wavelengths. This may also be the case for typical bedding materials. However, advantageously a difference between the skin reflection and bed reflection may start to occur at around 905 nm, which can be attributed to water absorption of the skin which is typically absent in the bedding. Hence, even in the challenging background with a white pillow (i.e., the color of the white pillow may be similar to that of skin in NIR), the weighting maps may still be used to discriminate skin and pillow using water absorption contrast at least at 905 nm.

Fig. 9 shows a sequence of images and generated weighting maps from the first four eigenvectors (Eig.1 to Eig. 4). It has been found that regardless of the posture and position of the subject, the weighting maps are consistently biasing the image by attenuating similar parts of the scene. The first row in Fig. 9 shows a sequence of RGB images over time. The second to fifth row indicate the generated weighting maps corresponding to the respective image in the first row.

During a first period denoted by P1 the subject is lying in bed facing the camera. In such a situation, also conventional face detection may provide good results. In the second phase indicated by P2 the subject performs a motion by turning to the side. Hence, transitioning into a position where conventional face detection may fail. It should further be noted that the weighting map in the penultimate row not only correctly identifies a face region but also correctly detects the hand 25 of the subject which can therefore provide additional valuable input for determining the physiological parameter of the subject. During period P3 the subject assumes a position lying on the side. During period P4 the subject leaves the bed. In such a situation, conventional approaches that rely on tracking a region-of-interest may fail once the subject has left the field of view of the camera. However, as shown in period P5, the approach proposed herein correctly resumes operation and can again correctly weight the skin portions of the subject as e.g. indicated by the weighting map based on the third eigenvector Eig.3.

Reference is now made to an advantageous combination with the second aspect of the present disclosure wherein a statistical parameter value indicative of a statistical dispersion of pixel values of an image frame is evaluated. The conventional way of combining image pixel values into a single value is spatial averaging, i.e., the calculation of a central tendency metric. However, it only works when the weighted RGB image where the correct skin-pixels are provided with an increased weight. In case the weighted image frame is polluted by non-skin pixels, such averaging may provide inaccurate results. The inventors have recognized that problems and disadvantages of the prior art relating to an averaging of pixels can be mitigated by computing a statistical property characterizing or indicative of a statistical dispersion, such as the variance or standard deviation, of pixels of the weighted image frame to combine the weighted pixel values and using this statistical parameter value in determining a candidate signal based on which a physiological parameter value of the subject is extracted. As mentioned above, a pixel as used herein can also refer to the down-sampled image pixels or patches.

The rationale of using a statistical parameter value indicative of a statistical dispersion of the pixel values such as the variance is the following: When the non-skin pixels dominate the weighted image, they dominate the mean. Subtracting the mean and measuring the additional variation may thus reduce the impact of the non-skin pixels. Further details will explained further below. Based on this understanding it had been recognized that the variance will be less effective when the skin and non-skin pixels have similar DC-color. Nevertheless, it can be seen as an alternative or back-up for the mean, especially in the case of imperfect weighting maps, where both the skin and known skin regions are emphasized.

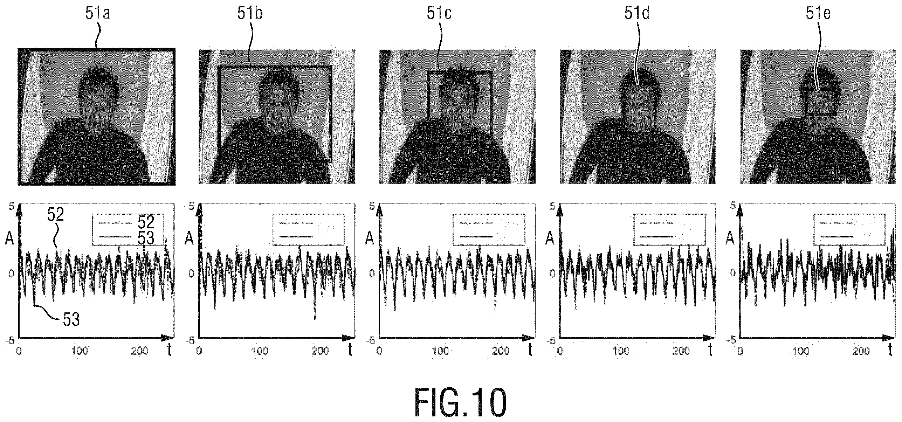

Figs. 10 illustrates that mean and variance can have complementary strength when combining pixels from different weighted RBG images using different regions. The top row illustrates the same image frame, wherein different regions that form the basis for the combination of pixel values are indicated by the frames 51a-51e. The graphs in the bottom row provide the extracted candidate signals that are extracted from the regions as indicated by the frames 51a-51e, respectively. In each of the graphs in the bottom row, the horizontal axis denotes the time t, given by the frame number, wherein the vertical axis denotes an amplitude of the extracted candidate signal.

For each of said image frames, two statistical parameter values are determined form the regions indicated by frames 5 1a to 5 1e. The first statistical parameter value is indicative of a statistical dispersion of pixel values within said frame. The second statistical parameter value is indicative of a central tendency of pixel values within said frame. The respective statistical parameter values are concatenated over time based on the time-sequence of the corresponding image frames to obtain a first and a second candidate signal. In the given example, the first candidate signal 53 corresponds to the standard deviation as a parameter indicative of the statistical dispersion, whereas the second candidate signal 52 corresponds to the mean as a central tendency metric.

More precisely, in the given example, the mean-based candidate signal 52 and the variance-based signal 53 are generated by mean (R/G) and var (R/G) respectively, wherein R/G is a ratio of the pixel values in the red and green color channels of an RGB image. For a visualization purpose, both the mean signal and variant signal have low frequency components (< 40 bpm) removed, mean subtracted and standard deviation normalized. It has been found that the mean-based candidate signal 52 and the variance-based candidate signal 53 show complementary strength. When non-skin pixels dominate, as in 51a, the variance-based candidate signal 53 is advantageous, whereas when skin pixels dominate, as in 51e, the mean-based candidate signal 52 performs better. Hence, it has been found that evaluating a statistical parameter value indicative of statistical dispersion can provide better performance in case of a polluted image frame with non-skin pixels.

Referring again to Fig. 7, step 105 this can be used to determine, for each weighted image frame $J_i$ weighted by a corresponding weighting map $\overline{W}_i$ a first statistical parameter value indicative of a statistical dispersion of pixel values of said weighted image frame and a second statistical parameter value indicative of a central tendency of pixel values of said weighted image frame. The output of step 105 are thus two statistical parameter values. The respective statistical parameter values can be concatenated over time based on the time-sequence of the corresponding weighted image frames to obtain a first candidate signal 107 based on said first statistical parameter values, a second candidate signal 107' based on said second statistical parameter values. Correspondingly, additional weighted image frames can be processed accordingly, as indicated by step 106, to obtain further candidate signals 108, 108'.

The respective candidate signals over time wherein e.g. mean and variance may have complementary strengths as indicated in Fig. 10, can be written as:

$$\begin{cases} T_{2i-1}(c,t) & = \mathbf{mean}(\mathbf{J_i}(x,c,t)) \\ T_{2i}(c,t) & = \mathbf{var}(\mathbf{J_i}(x,c,t)) \end{cases}, \qquad (8)$$

where $T_i$ (c, t) denotes a candidate signal; **mean** (·) denotes an averaging operator; **var** (·) denotes the variance operator. Hence, in view of the two different ways to determining and concatenating different statistical parameter values 107 and 107' as well as 108 and 108' in the example of Fig. 7, the number of temporal traces is double the number of weighting maps.

Referring again to Fig. 7, in the next step 109, a physiological parameter can be extracted based on said candidate signals obtained in the previous step. To extract the physiological parameter from the candidate signals T, known algorithms used in photoplethysmography can be applied. Exemplary algorithms are: HUE *(G. R. Tsouri and Z. Li, "On the benefits of alternative color spaces for noncontact heart rate measurements using standard red-green-blue cameras", J. Biomed. Opt., vol. 20, no. 4, p. 048002, 2015); PCA (M. Lewandowska et al., "Measuring pulse rate with a webcam - a non-contact method for evaluating cardiac activity ", in Proc. Federated Conf. Comput. Sci. Inform. Syst. (FedCSIS), pp. 405-410, 2011); ICA (M.-Z. Poh et al., "Advancements in noncontact, multiparameter physiological measurements using a webcam ", IEEE Trans. Biomed. Eng., vol. 58, no. 1, pp. 7-11, 2011); CHROM (G. de Haan and V. Jeanne, "Robust pulse rate from chrominance-based rPPG", IEEE Trans. Biomed. Eng., vol. 60, no. 10, pp. 2878-2886, 2013); PBV (G. de Haan and A. van Leest, "Improved motion robustness of remote-PPG by using the blood volume pulse signature", Physiol. Meas., vol. 35, no. 9, pp. 1913-1922, 2014); ABPV (M. van Gastel, S. Stuijk and G. de Haan, "New principle for measuring arterial blood oxygenation, enabling motion-robust remote monitoring", Nature Scientific Reports, p. 1- 16 19 2016); and POS (W. Wang et al., "Algorithmic principles of remote-PPG", IEEE Trans. Biomed. Eng., vol. PP, no. 99, 2016).*

The extraction of a remote-PPG signal or physiological parameter from each of the candidate signals $T_i$ can generally be expressed as:

$$\mathbf{P_i} = \mathrm{rPPG}(\mathbf{T_i}), \qquad (9)$$

where rPPG $(\cdot)$ denotes a core rPPG function, i.e. an algorithm for extracting a physiological parameter of the subject, such as the pulse, from the input candidate signal.

Optionally, further processing steps can be applied in order to determine a most likely pulse-signal from the candidate pulse signals $P_i$. For example, due to the use of both a central tendency (e.g. the mean) and a dispersion-related measure (e.g. the variance) for the spatial pixel combination, multiple $T_i$ (and thus $P_i$) may contain useful pulsatile content. Therefore, it would be advantageous to combine several candidate signals instead of selecting just one of them. Advantageously, for the case of extracting a pulse of the subject as the physiological parameter of the subject, only pulsatile frequency components in $P_i$ may be of interest such that it is proposed to combine frequency components from a different candidate signals instead of directly combining (time) signals.

Advantageously, in order to arrive at a clean output, higher weights can be given to components that are more likely to be pulse-related during the combination. However it has to be considered that the frequency amplitude may not directly be used to determine the weights or selected the components, because a large amplitude may not be due to pulse but due to motion artifacts. In view of a relation of pulsatile energy and motion energy it is thus proposed to estimate an intensity signal of each $T_i$ and use an energy ratio between pulsatile components and intensity components as weights. The rationale is: if a frequency component in $P_i$ is caused by pulse, it should have larger pulsatile energy with respect to the total intensity energy. If a component has balanced pulsatile and intensity energies, its "pulsatile energy" is more likely to be noise/motion induced. It should be mentioned that the use of intensity signal here is mainly for suppressing the background components although it may suppress motion artifacts as well.

The extraction of an intensity-signal from each $T_i$ can be expressed as:

$$\mathbf{Z_i} = \sum_{c=1}^{3} \mathbf{T_i}(c), \qquad (10)$$

which is basically the summation of the R, G and B feature signals in case of an RBG signal. Since a local spectral energy contrast between the components in $P_i$ and $Z_i$ is used to derive their combining weights, their total energy (i.e., standard deviation) is first normalized and then they are transformed into the frequency domain e.g. using the Discrete Fourier Transform (DFT):

$$\begin{cases} \mathbf{Fp_i} = \mathbf{DFT}(\frac{\mathbf{P_i} - \mu(\mathbf{P_i})}{\sigma(\mathbf{P_i})}) \\ \mathbf{Fz_i} = \mathbf{DFT}(\frac{\mathbf{Z_i} - \mu(\mathbf{Z_i})}{\sigma(\mathbf{Z_i})}) \end{cases}, \qquad (11)$$

where DFT $(\cdot)$ denotes the DFT operator. A weight for b-th frequency component in $\mathbf{Fp_i}$ can be derived by:

$$W_{i,b} = \begin{cases} \dfrac{\mathbf{abs}(Fp_{i,b})}{1 + \mathbf{abs}(Fz_{i,b})}, & \text{if } b \in \mathbf{B} = [b_1, b_2], \\ 0, & \text{elsewhere,} \end{cases} \qquad (12)$$

where **abs** $(\cdot)$ takes the absolute value (i.e. amplitude) of a complex value; **B** optionally denotes a band for filtering such as a heart-rate and for eliminating clearly non-pulsatile components which can e.g. be defined as [40, 240] beats per minute (bpm) according to the resolution of $\mathbf{Fp_i}$. Optionally an additive component in the denominator, here +1, is provided which prevents boosting of noise when dividing a very small value, i.e., the total energy is 1 after the normalization in (11). Afterwards, the weighting vector $W_i = [W_{i,\,1}, W_{i,\,2}, ..., W_{i,n}]$ can be used to weight and combine $Fp_i$ as:

$$\mathbf{Fh} = \sum_{i=1}^{4K} \mathbf{W_i} \odot \mathbf{Fp_i}. \qquad (13)$$

The combined frequency spectrum Fh can further be transformed back to the time domain, e.g. using the Inverse Discrete Fourier Transform (IDFT):

$$\mathbf{h} = \mathbf{IDFT}(\mathbf{Fh}), \qquad (14)$$

where **IDFT** $(\cdot)$ denotes the Inverse Discrete Fourier Transform operator. Consequently, a long-term pulse-signal or physiological parameter signal H can be derived by concatenating sections h, e.g. by overlap-adding h, (preferably after removing its mean and normalizing its standard deviation) estimated in different time windows or short sequences, e.g., using a sliding window The physiological parameter of the subject, such as the pulse rate, can then be extracted based thereon and provided as an output in step 110 of Fig. 7.

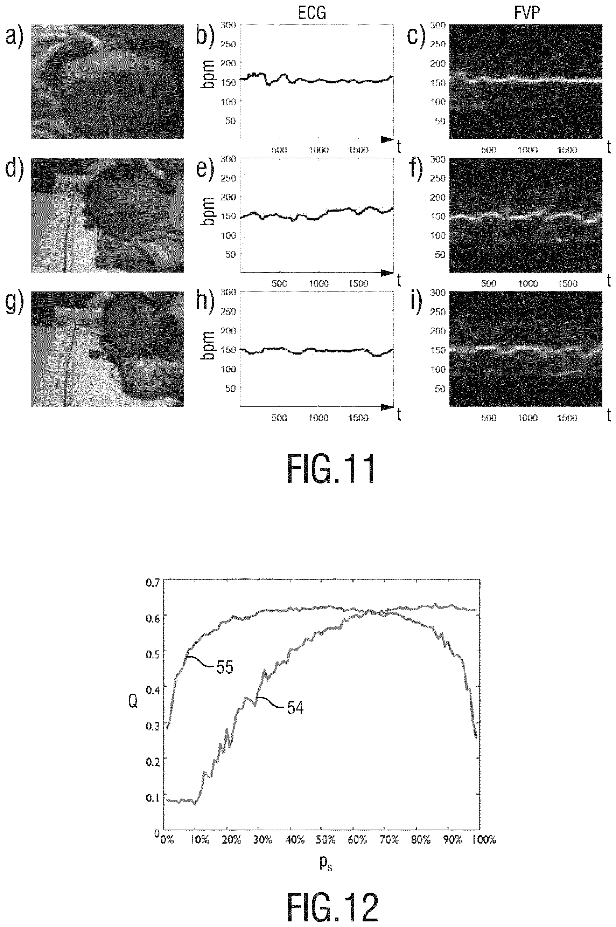

Fig. 11 shows a comparison of the performance of the system provided herein with a contact-based conventional ECG-based system. The graphs in the first column denoted by (a), (d), (g) show exemplary image frames acquired by an RGB camera in a neonatal intensive care unit (NICU). As can be seen, the baby shows significant movement between the image frames. Supply hoses render the detection of facial features difficult. The second row with graphs (b), (e), (h) shows the pulse of the baby acquired by a conventional contact-based electrocardiogram (ECG). The horizontal axis denotes the time t in frames of the imaging unit whereas the vertical axis denotes the pulse rate in beats per minute (bpm). The third column of graphs indicated by (c), (f), (i) shows graphs wherein the pulse rate is determined using the method as proposed herein, in particular as described in detail with reference to Fig. 7. The method is referred to as full video pulse-extraction (FVP). As can be seen from the comparison of the second and third column, the contactless method disclosed herein shows very good correspondence with the ground truth (ECG) contact-based measurement.

Fig. 12 shows a graph relating to a performance improvement that can be achieved by the second aspect of the present disclosure. The horizontal axis denotes a percentage of skin pixels $p_s$ of an image frame from which the physiological parameter value is extracted; whereas the vertical axis denotes a quality measure of the extracted signal.

Referring to Fig. 10, the frame 51a comprises a low percentage of skin pixels whereas the frame 51e provides a high percentage of skin pixels. In Fig. 12, the curve 55 denotes a quality regarding a candidate signal based on a first statistical parameter indicative of a statistical dispersion of pixel values of said frame, here the variance (cf. trace 53 in Fig. 10). The curve 54, on the other hand, denotes a quality regarding a candidate signal based on a second statistical parameter value indicative of a central tendency of pixel values of said image frames, here in a mean of the pixel values of the respective frame (cf. trace 52 in Fig. 10). As can be seen from Fig. 12 and also explained with reference to Fig. 10, the two curves 54 and 55 have complementary strength, wherein the mean-based signal 54 is advantageous when a high percentage of skin pixels is provided (corresponding to good ROI selection or accurate weighting according to the first aspect of the present disclosure), whereas the evaluation based on the variance denoted by curve 55 shows advantageous performance in case of a polluted signal comprising a high percentage of non-skin pixels. Hence, dispersion can be particularly helpful in case the image frame is polluted by a significant number of non-skin pixels. However, since it cannot be assumed that non-skin pixels will always be present, in an advantageous embodiment both a first dispersion-based candidate signal and a second candidate signal based on a central tendency can be evaluated.

By extracting candidate signals using both techniques, or both statistical metrics, during the extraction step the candidate signal providing the best signal quality can be evaluated. In case multiple wavelength channels are available a more robust approach for extracting a physiological signal can optionally be used. Examples have been shown in the literature and include: a fixed weighted sum over candidate signals of different wavelength channels (RGB, NIR), CHROM, POS, PBV-method, ABPV-method, blind source separation (PCA, ICA) preferably after normalizing the candidate signals, in particular when the candidate signals are based on the central tendency of pixels, e.g. by dividing by their temporal mean, or taking their logarithm and offset removal.

In case a dispersion metric is used to provide the (concatenated) candidate signal, relative pulsatilities in different wavelength channels may be affected by a distribution of pixels. For blind source separation methods this may not be a problem, but for methods that make assumption on, either a direction in color-space of the distortions, or of the PPG-signal (or other physiological parameter signal) itself it is advantageous to correct the candidate signals with respect to gain or sign, prior to extraction. More particularly, said correction may be based on a first principal component of pixel values. For example this can be based on a vector that points from a color point of skin color to a color point of background of pixel values in particular in a region-of-interest or highly weighted region.

It has been found that, in case of using multiple color channels, the use of statistical dispersion metrics may change a relative amplitude between different concatenated color traces. Hence, it may render the rPPG extraction methods such as PBV, ABPV, CHROM, or POS, using blood volume pulse priors invalid or sub-optimal. Several different ways of correction can be used, optionally even in parallel: (i) Use of principal component analysis (PCA) to correct a relative amplitude of different color channels (or candidate signals obtained based on different color channels). Alternatively, a PBV vector of PBV, a set of PBV-vectors in ABPV, or a projection-axes of CHROM/POS can be corrected. Thereby, prior-related rPPG methods can be used. (b) Use of weighting maps, in particular in accordance with the first aspect of the present disclosure, to suppress non-skin pixels (similar to "pulling non-skin pixels to black"). Thereby, prior-related rPPG methods can be used. (c) Use the blind source separation (BSS) extraction. Thereby, amplitude-correction or pixel-weighting are not necessarily needed. (d) Combine the multi-wavelength images into a single-wavelength image (for example by 2log(G)-log(R)-log(B)) in a preprocessing step and then use a dispersion metric to combine spatial pixels.

Advantageously such a correction that does not have to be measured every frame, but only e.g. once in a predetermined analysis window. Sliding window processing can optionally be applied. Such a vector may be temporarily filtered e.g. recursively, to stabilize the correction. Gain correction may comprise dividing the color channels by their respective component of said principal component. For example, such a connection based on (a first) principal component of pixel values may assume that next to the skin pixels a single background color occurs in a region-of-interest.

In a complex situation, however, several different background colors may occur. In such a case such a correction may not be correct, since there is no single different background color. Optionally, a weighting can thus be applied, wherein non-skin pixels in the background are attenuated based on a likelihood of being skin pixels. Preferably pixels can be pulled towards black or white the more likely they do not belong to the skin. This causes multiple colored background patches to concentrate near a single color point, such as white or black in the given example, which can make the aforementioned correction valid again.

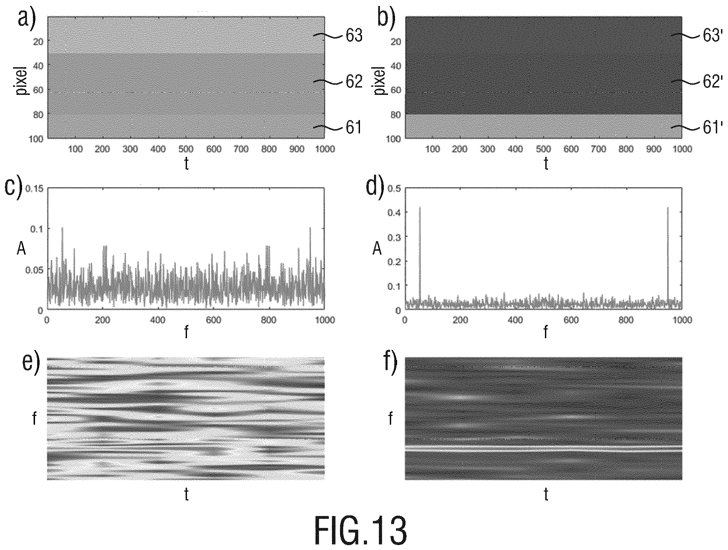

An exemplary embodiment of this process is illustrated in Fig. 13. The left column in Fig. 13 refers to an exemplary situation wherein a region-of-interest (ROI) phantom is provided having skin pixels 61 as well as differently colored non-skin regions 62 and 63. The right column refers to the same ROI phantom however with correction applied, wherein non-skin pixels in regions 62' and 63' are attenuated. As can be seen from the second row, the ROI phantom without weighting applied shows a distorted spectrum having a low quality factor in Fig. 13 (c), whereas the spectrum with the weighting applied shows very distinct frequency peaks in Fig. 13 (d). The horizontal axis in Fig. 13(c) and (d) denotes the frequency, whereas the vertical axis denotes the amplitude A.

Fig. 13 (e) and (f) show the corresponding spectrograms wherein the horizontal axis denotes the time t and the vertical axis denotes the frequency f. As can be seen from the comparison of Fig. 13 (e) and (f), the weighting by attenuating non-skin pixels leads to a very clean spectrogram wherein the pulsatile signal component can be clearly distinguished.

Referring again to the advantageous combination of the first and the second aspect of the present disclosure, it should be noticed that in many practical situations it can be difficult to make a clear cut distinction between skin and non-skin parts in an image. Typically, setting a high specificity results in loss of many pixels or areas that could have provided valuable information, but setting a high sensitivity for skin typically results in many non-skin areas thereby diluting the information of interest. This incapacity of striking a correct balance is an underlying notion in the map generation as it simply refutes the idea of hard boundaries. A consequence is that the signal processing needs to handle both relatively clean signals in particular due to proper weighting maps on the one hand and rather polluted situations on the other hand. This ability can be attained by the second aspect of the present disclosure by advantageously evaluating both a first statistical parameter value indicative of a statistical dispersion of pixel values of said image frame and a second statistical parameter value indicative of a central tendency of pixel values. In the non-limiting examples described herein, the mean as a central tendency measure and variance as a dispersion-related measure will be evaluated. The commonly used approaches only use the mean as source of information only. As will be shown below in more detail below and as also indicated by Fig. 12, the mean and variance have complementary strength. To simplify the illustration reference will be made to a single channel case. However, conclusions carry over to multi-channel situations.

In considering the task of pulse extraction, each pixel in an image may be described as either skin or non-skin. Thus, there can be skin and non-skin distributions in an image. Two statistical models can be assumed, for either case, with a probability density function, PDF $p_o(x)$, and associated mean $\mu_o$ and standard deviation $\sigma_o$ where x denotes the signal strength (color intensity) and o is either skin s or background b. It can furthermore be supposed that the full image has a fraction $f_o$ of either pixels (implying $f_s + f_b = 1$). The composite image pixel PDF p(x) can be written as:

$$p(x) = f_s \cdot p_s(x) + f_b \cdot p_b(x). \qquad (15)$$

The mean of p(x) is:

$$\mu = \mathbb{E}[p(x)] = f_s \cdot \mathbb{E}[p_s(x)] + f_b \cdot \mathbb{E}[p_b(x)] \\ = f_s \cdot \mu_s + f_b \cdot \mu_b \qquad (16)$$

where $\mathbb{E}[\cdot]$ denotes the expectation. The variance of p (x) is:

$$\sigma^2 = \mathbb{E}[p^2(x)] - (\mathbb{E}[p(x)])^2 \\ = f_s \cdot \mathbb{E}[p_s^2(x)] + f_b \cdot \mathbb{E}[p_b^2(x)] - (f_s \cdot \mu_s + f_b \cdot \mu_b)^2. \qquad (17)$$

It is known that for $p_o$ (x) (including the skin and non-skin), $\mathbb{E}[p_o(x)] = \mu_o$ and $\mathbb{E}[(p_o(x) - \mu_o)^2] = \sigma_o^2$.

Thus $\mathbb{E}[p_o^2(x)]$ can be expressed as

$$\mathbb{E}[p_o^2(x)] = \mathbb{E}[(p_o(x) - \mu_o + \mu_o)^2] \\ = \mathbb{E}[(p_o(x) - \mu_o)^2] + 2\mu_o \cdot \mathbb{E}[p_o(x) - \mu_o] + \mathbb{E}[\mu_o^2] \\ = \sigma_o^2 + \mu_o^2. \qquad (18)$$

Therefore, [17] can be rewritten as:

$$\sigma^2 = f_s \cdot (\sigma_s^2 + \mu_s^2) + f_b \cdot (\sigma_b^2 + \mu_b^2) - (f_s \cdot \mu_s + f_b \cdot \mu_b)^2 \\ = f_s \cdot \sigma_s^2 + f_b \cdot \sigma_b^2 + f_s \cdot \mu_s^2 + f_b \cdot \mu_b^2 - (f_s \cdot \mu_s + f_b \cdot \mu_b)^2. \\ = f_s \cdot \sigma_s^2 + f_b \cdot \sigma_b^2 + f_s \cdot f_b \cdot (\mu_s - \mu_b)^2 \qquad (19)$$

Now it can be assumed that the mean skin-level is modulated for example by the blood perfusion. $\mu_s$ can be expressed as a combination of a steady DC-component and a time-dependent AC-component:

$$\mu_s = \bar{\mu}_s + \tilde{\mu}(t), \qquad (20)$$

where $\overline{\mu_s}$ is the steady DC component and $\tilde{\mu}$ is the time-varying AC component. Furthermore it can be assumed that the background statistics are constant (i.e. assuming means such as a weighting mask to attenuate the background) and neglecting all modulations in the variance of the skin. Therefore, the full image mean in (16) can be rewritten as:

$$\mu = f_s \cdot (\bar{\mu}_s + \tilde{\mu}(t)) + f_b \cdot \mu_b \\ = f_s \cdot \bar{\mu}_s + f_b \cdot \mu_b + f_s \cdot \tilde{\mu}(t), \qquad (21)$$

and the full (weighted) image variance in (19) can be rewritten as:

$$\sigma^2 = f_s \cdot \sigma_s^2 + f_b \cdot \sigma_b^2 + f_s \cdot f_b \cdot (\bar{\mu}_s + \tilde{\mu}(t) - \mu_b)^2$$
$$= f_s \cdot \sigma_s^2 + f_b \cdot \sigma_b^2 + f_s \cdot f_b \cdot ((\bar{\mu}_s - \mu_b)^2 +$$
$$2(\bar{\mu}_s - \mu_b) \cdot \tilde{\mu}(t) + \tilde{\mu}^2(t)) \qquad (22)$$
$$\approx f_s \cdot \sigma_s^2 + f_b \cdot \sigma_b^2 + f_s \cdot f_b \cdot (\bar{\mu}_s - \mu_b)^2 +$$
$$2f_s \cdot f_b \cdot (\bar{\mu}_s - \mu_b) \cdot \tilde{\mu}(t)$$

where $\tilde{\mu}^2$ can be ignored in the approximation, as the squared pulsatile changes are orders of magnitude smaller than other DC-related components. Consequently, it can be found that the pulsatile component in the full (weighted) image mean and full (weighted) image variance are respectively provided by:

$$\begin{cases} \hat{\mu} = f_s \cdot \tilde{\mu}(t) \\ \hat{\sigma}^2 = 2f_s \cdot (1 - f_s) \cdot (\bar{\mu}_s - \mu_b) \cdot \tilde{\mu}(t), \end{cases} \qquad (23)$$

As expected, if $f_s = 0$ (no skin), there is no pulsatile component in either statistical variable. Furthermore it can be observed that the pulse-contribution to the mean is a linearly decreasing function of $f_s$, i.e., the fraction of skin-pixels.

In other words, with less skin-pixels also less pulsatile amplitude is contained in the mean. The variance shows another behavior as the function of the skin fraction. It contains no pulsatile component in both extreme cases (all skin or all background) but peaks in the middle assuming at least some contrast between skin and background: $\overline{\mu}_s - \mu_b \neq 0$.

This is also reflected by Fig. 12 by trace 55. The previous indicates that dependent on the fraction $f_s$ and contrast, there may be more pulsatile information in the variance than in the mean. This is actually the underlying explanation of the findings illustrated in Fig. 12. When the region-of-interest is dominated by skin-pixels, the mean signal reflects the blood volume changes in a better way (i.e. the signal is less noisy). When the region-of-interest contains certain amount of non-skin pixels, the variance signal shows much clear pulsatile variations. Therefore, the use of variance as a dispersion-related measure, in particular in addition to the mean, can be valuable since it cannot be safely assumed that the image frame only contains skin.

In conclusion, the present disclosure provides an advantageous device system and method for determining a physiological parameter of a subject. In particular, according to the first aspect of the present disclosure the need for a region-of-interest initialization/detection and tracking can be eliminated. The second aspect of the present disclosure further provides an improved signal in case of a polluted input signal wherein the image frames or portions selected thereof or highly weighted therein comprise a combination of skin pixels and non-skin pixels.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single processor, element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1.   Device (10) for determining a physiological parameter of a subject (20), the device comprising:

   - an interface (11) for receiving image data of a scene, said image data comprising a time-sequence of image frames; and
   - a processor (12) for processing said image data, wherein the processor is configured to perform the steps of: weighting pixel values of the image frames by reducing a weight of non-skin pixels relative to skin pixels;
   - determining, for each of said image frames, a first statistical parameter value indicative of a statistical dispersion of pixel values of said image frame (202);

- concatenating said first statistical parameter values over time based on the time-sequence of the corresponding image frames to obtain a first candidate signal (203);
- extracting a physiological parameter of the subject based on said first candidate signal (204).

2. Device as claimed in claim 1, wherein said first statistical parameter value is indicative of at least one of a standard deviation, a variance, mean absolute difference, median absolute difference and/or an interquartile range.

3. Device as claimed in claim 1 or 2, wherein the processor (12) is configured to perform the steps of:

- determining, for each of said image frames, a second statistical parameter value indicative of a central tendency of pixel values of said image frame;
- concatenating said second statistical parameter values over time based on the time-sequence of the corresponding image frames to obtain a second candidate signal; and
- extracting a physiological parameter of the subject based on said first and/or second candidate signal.

4. Device as claimed in claim 3, wherein the processor is configured to extract said physiological parameter of the subject based on the second candidate signal, wherein the extraction of the physiological parameter of the subject based on the second candidate signal is further supported by the first candidate signal.

5. Device as claimed in any preceding claim, wherein the processor (12) is further configured to determine the first statistical parameter value based on a logarithm of pixel values.

6. Device as claimed in any preceding claim, wherein the processor (12) is further configured to perform a preprocessing step of selecting a region-of-interest by selecting pixels of an image frame based on a local property of the image indicative of a skin of the subject (20) and to determine said first statistical parameter value indicative of a statistical dispersion based on pixel values from said region-of-interest.

7. Device as claimed in any preceding claim, wherein said step of extracting the physiological parameter comprises a correction, in particular based on a principal component of at least pixels of an image frame.

8. Device as claimed in claim 7, wherein the extracting of the physiological parameter is based on a pulse-blood-volume (PBV) extraction method and wherein said correction based on a principal component value is applied to a PBV-signature vector.

9. Device as claimed in any preceding claim, wherein the processor (12) is configured to determine at least the first candidate signal and a second candidate signal and wherein the step of extracting the physiological parameter of the subject (204) further comprises the step of selecting at least one candidate signal based on a quality metric.

10. Device as claimed in any preceding claim, wherein the processor (12) is further configured to determine at least the first candidate signal and a second candidate signal and to apply a blind source separation technique to the candidate signals to obtain independent signals and to select at least one of said independent signals based on a quality metric.

11. Device as claimed in any preceding claim, wherein the processor (12) is configured to determine at least the first candidate signal and a second candidate signal and to combine at least two of said candidate signals in the frequency domain.

12. System (1) for determining a physiological parameter of a subject (20); the system comprising:

- imaging unit (2) configured to acquire image data of a scene, said image data comprising a time-sequence of image frames; and
- a device (10) for determining a physiological parameter of a subject (20) as claimed in any preceding claim based on the acquired image data.

13. A method (200) for determining a physiological parameter of a subject (20), the method comprising the steps of:

- receiving image data of a scene, said image data comprising a time-sequence of image frames (201); weighting pixel values of the image frames by reducing a weight of non-skin pixels relative to skin pixels;
- determining, for each of said image frames, a first statistical parameter value indicative of a statistical dispersion of pixel values of said image frame (202);
- concatenating said first statistical parameter values over time based on the time-sequence of the corresponding image frames to obtain a first candidate signal (203);
- extracting a physiological parameter of the subject based on said first candidate signal (204).

14. Computer program comprising program code means for causing a computer to carry out the steps of the method (200) as claimed in claim 13 when said computer program is carried out on the computer.

**Patentansprüche**

1. Vorrichtung (10) zur Bestimmung eines physiologischen Parameters einer Person (20), die Vorrichtung besteht aus:

    - einer Schnittstelle (11) zum Empfangen von Bilddaten einer Szene, wobei die besagten Bilddaten eine zeitliche Abfolge von Einzelbildern umfassen; und
    - einem Prozessor (12) zum Verarbeiten der Bilddaten, wobei der Prozessor konfiguriert ist, die folgenden Schritte auszuführen:
    Gewichten von Pixelwerten der Bilder durch Reduzieren einer Gewichtung von Nicht-Haut-Pixeln relativ zu Hautpixeln;
    - Bestimmen eines ersten statistischen Parameterwerts für jeden der besagten Bilder, der eine statistische Streuung von Pixelwerten des besagten Bildes (202) anzeigt;
    - Verketten der besagten ersten statistischen Parameterwerte über die Zeit, basierend auf der zeitlichen Abfolge der entsprechenden Einzelbilder, um ein erstes Kandidatensignal (203) zu erhalten;
    - Extrahieren eines physiologischen Parameters der Person, basierend auf dem besagten ersten Kandidatensignal (204).

2. Vorrichtung nach Anspruch 1, wobei der besagte erste statistische Parameterwert mindestens eines von einer Standardabweichung, einer Varianz, einer mittleren absoluten Differenz, einer mittleren absoluten Differenz und/oder einem Interquartilsabstand anzeigt.

3. Vorrichtung nach Anspruch 1 oder 2, wobei der Prozessor (12) dazu konfiguriert ist, die folgenden Schritte auszuführen:

    - Bestimmen eines zweiten statistischen Parameterwerts für jeden der besagten Bilder, der eine zentrale Tendenz von Pixelwerten des besagten Bildes anzeigt;
    - Verketten der besagten zweiten statistischen Parameterwerte über die Zeit, basierend auf der zeitlichen Abfolge der entsprechenden Einzelbilder, um ein zweites Kandidatensignal zu erhalten; und
    - Extrahieren eines physiologischen Parameters der Person, basierend auf dem besagten ersten und/oder zweiten Kandidatensignal.

4. Vorrichtung nach Anspruch 3, wobei der Prozessor dazu konfiguriert ist, besagte physiologische Parameter der Person auf der Grundlage des zweiten Kandidatensignals zu extrahieren, wobei die Extraktion der physiologischen Parameter der Person auf der Grundlage des zweiten Kandidatensignals durch das erste Kandidatensignal zusätzlich unterstützt wird.

5. Vorrichtung nach einem der vorhergehenden Ansprüche, wobei der Prozessor (12) ferner konfiguriert ist zum Bestimmen des ersten statistischen Parameterwerts basierend auf einem Logarithmus der Pixelwerte.

6. Vorrichtung nach einem der vorhergehenden Ansprüche, wobei der Prozessor (12) ferner konfiguriert ist zum Durchführen eines Vorverarbeitungsschritts zum Auswählen eines interessierenden Bereichs durch Auswählen von Pixeln eines Bildes, basierend auf einer lokalen Eigenschaft des Bildes, die eine Haut der Person (20) anzeigt, und um den besagten ersten statistischen Parameterwert zu bestimmen, der eine statistische Streuung basierend auf Pixelwerten aus dem interessierenden Bereich anzeigt.

7. Vorrichtung nach einem der vorhergehenden Ansprüche, wobei der besagte Schritt des Extrahierens der physiologischen Parameter eine Korrektur umfasst, insbesondere basierend auf einer Hauptkomponente von mindestens Pixeln eines Bildes.

8. Vorrichtung nach Anspruch 7, wobei das Extrahieren des physiologischen Parameters auf einem Pulse-Blood-Volume (PBV)-Extraktionsverfahren basiert und wobei besagte Korrektur, die auf einem Hauptkomponentenwert basiert, auf einen PBV-Signaturvektor angewendet wird.

9. Vorrichtung nach einem der vorhergehenden Ansprüche, wobei der Prozessor (12) dazu konfiguriert ist, mindestens das erste Kandidatensignal und ein zweites Kandidatensignal zu bestimmen und wobei der Schritt des Extrahierens des physiologischen Parameters der Person (204) weiterhin den Schritt des Auswählens mindestens eines Kandidatensignals, basierend auf einer Qualitätsmetrik, umfasst.

10. Vorrichtung nach einem der vorhergehenden Ansprüche, wobei der Prozessor (12) weiterhin dazu konfiguriert ist, mindestens das erste Kandidatensignal und ein zweites Kandidatensignal ferner konfiguriert zum Bestimmen mindestens des ersten Kandidatensignals und eines zweiten Kandidatensignals, um unabhängige Signale zu erhalten, und zum Auswählen mindestens eines der unabhängigen Signale, basierend auf einer Qualitätsmetrik.

11. Vorrichtung nach einem der vorhergehenden Ansprüche, wobei der Prozessor (12) dazu konfiguriert ist, mindestens das erste Kandidatensignal und ein zweites Kandidatensignal zu bestimmen und mindestens zwei der besagten Kandidatensignale im Frequenzbereich zu kombinieren.

12. System (1) zur Bestimmung eines physiologischen Parameters einer Person (20); das System umfasst:

   - Bildgebungseinheit (2), die dazu konfiguriert ist, Bilddaten einer Szene zu erfassen, wobei die besagten Bilddaten eine zeitliche Abfolge von Einzelbildern umfassen; und
   - Vorrichtung (10) zur Bestimmung eines physiologischen Parameters einer Person (20), wie beansprucht in einem der vorhergehenden Ansprüche, basierend auf den erfassten Bilddaten.

13. Verfahren (200) zur Bestimmung eines physiologischen Parameters einer Person (20), das Verfahren umfasst die folgenden Schritte:

   - Empfangen von Bilddaten einer Szene, wobei die Bilddaten eine zeitliche Abfolge von Bildern (201) umfassen; Gewichten von Pixelwerten der Bilder durch Reduzieren einer Gewichtung von Nicht-Haut-Pixeln relativ zu Hautpixeln;
   - Bestimmen eines ersten statistischen Parameterwerts für jedes der besagten Bilder, der eine statistische Streuung von Pixelwerten des besagten Bildes (202) anzeigt;
   - Verketten der besagten ersten statistischen Parameterwerte über die Zeit, basierend auf der zeitlichen Abfolge der entsprechenden Einzelbilder, um ein erstes Kandidatensignal (203) zu erhalten;
   - Extrahieren eines physiologischen Parameters der Person, basierend auf dem besagten ersten Kandidatensignal (204).

14. Computerprogramm, das Programmcodemittel umfasst, um einen Computer dazu zu veranlassen, die Schritte des Verfahrens (200) nach Anspruch 13 auszuführen, wenn das besagte Computerprogramm auf dem Computer ausgeführt wird.

## Revendications

1. Dispositif (10) pour déterminer un paramètre physiologique d'un sujet (20), le dispositif comprenant:

   - une interface (11) pour recevoir des données d'image d'une scène, lesdites données d'image comprenant une séquence temporelle d'images; et
   - un processeur (12) pour traiter lesdites données d'image, dans lequel le processeur est configuré pour effectuer les étapes de:
   Pondération des valeurs de pixel des images en réduisant un poids des pixels sans relation avec la peau par rapport aux pixels de la peau;
   - déterminer, pour chacune desdites images, une première valeur de paramètre statistique indicative d'une dispersion statistique des valeurs de pixel de ladite image (202);
   - enchaînement desdites premières valeurs de paramètres statistiques dans le temps sur la base de la séquence temporelle des images correspondantes pour obtenir un premier signal candidat (203);
   - extraire un paramètre physiologique du sujet sur la base dudit premier signal candidat (204).

**2.** Dispositif selon la revendication 1, dans lequel ladite première valeur de paramètre statistique est indicative d'au moins l'un des suivants, un écart type, une variance, une différence absolue moyenne, une différence médiane absolue et/ou un intervalle interquartile.

**3.** Dispositif selon la revendication 1 ou 2, dans lequel le processeur (12) est configuré pour effectuer les étapes de:

- déterminer, pour chacune desdites images, une deuxième valeur de paramètre statistique indicative d'une tendance centrale des valeurs de pixel de ladite image;
- enchaînement desdites deuxièmes valeurs de paramètres statistiques dans le temps sur la base de la séquence temporelle des images correspondantes pour obtenir un deuxième signal candidat;
- extraire un paramètre physiologique du sujet sur la base dudit premier et/ou deuxième signal candidat.

**4.** Dispositif selon la revendication 3, dans lequel le processeur est configuré pour extraire ledit paramètre physiologique du sujet étant basé sur le deuxième signal candidat, dans lequel l'extraction du paramètre physiologique du sujet sur la base du second signal candidat est en outre soutenu par le premier signal candidat.

**5.** Dispositif selon toute revendication précédente, dans lequel le processeur (12) est configuré en outre pour déterminer la première valeur de paramètre statistique sur la base d'un logarithme de valeurs de pixels.

**6.** Dispositif selon toute revendication précédente, dans lequel le processeur (12) est configuré en outre pour effectuer une étape de prétraitement consistant à sélectionner une région d'intérêt par sélection de pixels d'un cadre d'image sur la base d'une propriété locale de l'image indicative d'une peau du sujet (20) et pour déterminer ladite première valeur de paramètre statistique indicative d'une dispersion statistique basée sur les valeurs de pixel de ladite région d'intérêt.

**7.** Dispositif selon l'une quelconque des revendications précédentes, dans lequel ladite étape d'extraction du paramètre physiologique comprend une correction, notamment basée sur une composante principale d'au moins des pixels d'une image.

**8.** Dispositif selon la revendication 7, dans lequel l'extraction du paramètre physiologique est basée sur une méthode d'extraction de volume sanguin pulsé (PBV) et dans lequel ladite correction basée sur une valeur de composante principale est appliquée à un vecteur de signature PBV.

**9.** Dispositif selon toute revendication précédente, dans lequel le processeur (12) est configuré pour déterminer au moins le premier signal candidat et un deuxième signal candidat et dans lequel l'étape d'extraction du paramètre physiologique du sujet (204) comprend en outre l'étape de sélection d'au moins un signal candidat sur la base d'une mesure de qualité.

**10.** Dispositif selon toute revendication précédente, dans lequel le processeur (12) est configuré en outre pour déterminer au moins le premier signal candidat et un deuxième signal candidat et d'appliquer une technique de séparation de source aveugle aux signaux candidats pour obtenir des signaux indépendants et pour sélectionner au moins l'un desdits signaux indépendants sur la base d'une mesure de qualité.

**11.** Dispositif selon toute revendication précédente, dans lequel le processeur (12) est configuré pour déterminer au moins le premier signal candidat et un deuxième signal candidat et pour combiner au moins deux desdits signaux candidats dans le domaine fréquentiel.

**12.** Système (1) pour déterminer un paramètre physiologique d'un sujet (20); le système comprend:

- une unité d'imagerie (2) configurée pour acquérir des données d'image d'une scène, lesdites données d'image comprenant une séquence temporelle d'images; et
- un dispositif (10) pour déterminer un paramètre physiologique d'un sujet (20), comme revendiqué par l'une quelconque des revendications précédentes sur la base des données d'image acquises.

**13.** Un procédé (200) pour déterminer un paramètre physiologique d'un sujet (20), le procédé comprend les étapes de:

- recevoir des données d'image d'une scène, lesdites données d'image comprenant une séquence temporelle d'images (201);

pondération des valeurs de pixel des images en réduisant un poids des pixels sans relation avec la peau par rapport aux pixels de la peau;
- déterminer, pour chacune desdites images, une première valeur de paramètre statistique indicative d'une dispersion statistique des valeurs de pixel de ladite image (202);
- enchaînement desdites premières valeurs de paramètres statistiques dans le temps sur base de la séquence temporelle des images correspondantes pour obtenir un premier signal candidat (203);
- extraire un paramètre physiologique du sujet sur la base dudit premier signal candidat (204).

14. Programme informatique comprenant des moyens de code de programme pour amener un ordinateur à effectuer les étapes du procédé (200) selon la revendication 13 lorsque ledit programme d'ordinateur est exécuté sur l'ordinateur.

FIG.1

FIG.2

FIG.3

105 105'
$$\mu_1(c,t_1) \quad \sigma_1(c,t_1)$$

105 105'
$$\mu_1(c,t_2) \quad \sigma_1(c,t_2)$$

$$\mu_1(c,t_n) \quad \sigma_1(c,t_n)$$

$$J_1(x,c,t_1) \quad 103$$

$$J_1(x,c,t_2) \quad 103$$

$$J_1(x,c,t_n)$$

=

=

=

$$\overline{W}_1(x,t_1) \quad 102$$

$$\overline{W}_1(x,t_2) \quad 102$$

$$\overline{W}_1(x,t_n)$$

$\odot$

$\odot$

$\odot$

$$I(x,c,t_1) \quad 101$$

$$I(x,c,t_2) \quad 101$$

..... $$I(x,c,t_n) \longrightarrow t$$

$\odot$

$\odot$

$\odot$

$$\overline{W}_2(x,t_1) \quad 102$$

$$\overline{W}_2(x,t_2) \quad 102$$

$$\overline{W}_2(x,t_n)$$

=

=

=

$$J_2(x,c,t_1) \quad 104$$

$$J_2(x,c,t_2) \quad 104$$

$$J_2(x,c,t_n)$$

$$\mu_2(c,t_1) \quad \sigma_2(c,t_1)$$

$$\mu_2(c,t_2) \quad \sigma_2(c,t_2)$$

$$\mu_2(c,t_n) \quad \sigma_2(c,t_n)$$

106 106'

106 106'

# FIG.4

200

201

202

203

204

FIG.5

FIG.6

FIG.7

FIG.8

FIG.9

FIG.10

EP 3 664 704 B1

FIG.11

FIG.12

FIG.13

EP 3 664 704 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014180660 A1
- WO 2018029123 A1
- WO 2011042839 A1
- US 20140276099 A1
- WO 2011127487 A2

### Non-patent literature cited in the description

- **VERKRUYSSE et al.** Remote plethysmographic imaging using ambient light. *Optics Express,* 22 December 2008, vol. 16 (26), 21434-21445
- **MOÇO et al.** Motion robust PPG-Imaging through color channel mapping. *Biomedical Optics Express,* April 2016, vol. 7 (5), 1737-1754
- On spectral clustering: Analysis and an algorithm. **A. Y. NG ; M. I. JORDAN ; Y. WEISS.** Advances In Neural Information Processing Systems. MIT Press, 2001, 849-856
- **G. R. TSOURI ; Z. LI.** On the benefits of alternative color spaces for noncontact heart rate measurements using standard red-green-blue cameras. *J. Biomed. Opt.,* 2015, vol. 20 (4), 048002
- **M. LEWANDOWSKA et al.** Measuring pulse rate with a webcam - a non-contact method for evaluating cardiac activity. *Proc. Federated Conf. Comput. Sci. Inform. Syst. (FedCSIS),* 2011, 405-410
- **M.-Z. POH et al.** Advancements in noncontact, multiparameter physiological measurements using a webcam. *IEEE Trans. Biomed. Eng.,* 2011, vol. 58 (1), 7-11
- **G. DE HAAN ; V. JEANNE.** Robust pulse rate from chrominance-based rPPG. *IEEE Trans. Biomed. Eng.,* 2013, vol. 60 (10), 2878-2886
- **G. DE HAAN ; A. VAN LEEST.** Improved motion robustness of remote-PPG by using the blood volume pulse signature. *Physiol. Meas.,* 2014, vol. 35 (9), 1913-1922
- **M. VAN GASTEL ; S. STUIJK ; G. DE HAAN.** New principle for measuring arterial blood oxygenation, enabling motion-robust remote monitoring. *Nature Scientific Reports,* 2016, 1-16, 19
- **W. WANG et al.** Algorithmic principles of remote-PPG. *IEEE Trans. Biomed. Eng.,* 2016, vol. PP (99